(19) Europäisches Patentamt
European Patent Office
Office européen des brevets

(11) Veröffentlichungsnummer: **0 298 371 B1**

# (12) EUROPÄISCHE PATENTSCHRIFT

(45) Veröffentlichungstag der Patentschrift: **02.10.91**

(51) Int. Cl.⁵: **C07D 249/12**, A01N 43/653

(21) Anmeldenummer: **88110463.2**

(22) Anmeldetag: **30.06.88**

Die Akte enthält technische Angaben, die nach dem Eingang der Anmeldung eingereicht wurden und die nicht in dieser Patentschrift enthalten sind.

## (54) Substituierte Triazolinone.

(30) Priorität: **10.07.87 DE 3722821**

(43) Veröffentlichungstag der Anmeldung:
**11.01.89 Patentblatt 89/02**

(45) Bekanntmachung des Hinweises auf die Patenterteilung:
**02.10.91 Patentblatt 91/40**

(84) Benannte Vertragsstaaten:
**BE CH DE FR GB IT LI NL**

(56) Entgegenhaltungen:
**EP-A- 0 140 194**
**EP-A- 0 146 279**
**DE-A- 2 707 801**

**Pflanzenschutz und Schädlingsbekämpfung, K.H. Büchel, S. 170, Thieme Verlag Stuttgart, 1977**

(73) Patentinhaber: **BAYER AG**

**W-5090 Leverkusen 1 Bayerwerk(DE)**

(72) Erfinder: **Lindig, Markus, Dr.**
**Dahlienweg 16**
**W-4010 Hilden(DE)**
Erfinder: **Dickoré, Karlfried , Dr.**
**Nicolai-Hartmann-Strasse 19**
**W-5090 Leverkusen(DE)**
Erfinder: **Findeisen, Kurt, Dr.**
**In der Follmuehle 10**
**W-5068 Odenthal 2(DE)**
Erfinder: **Santel, Hans-Joachim, Dr.**
**Gruenstrasse 9a**
**W-5090 Leverkusen(DE)**
Erfinder: **Schmidt, Robert R., Dr.**
**Im Waldwinkel 110**
**W-5060 Bergisch Gladbach 2(DE)**
Erfinder: **Strang, Harry, Dr.**
**Unterdorfstrasse 6a**
**W-4000 Düsseldorf 31(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft neue substituierte Triazolinone, mehrere Verfahren zu ihrer Herstellung und ihre Verwendung als Herbizide.

Es ist bekannt, daß bestimmte Stickstoffheterocyclen wie beispielsweise das Imidazolidin-2-on-1-carbonsäureisobutylamid herbizide Eigenschaften besitzen (vgl. z. B. K. H. Büchel "Pflanzenschutz und Schädlingsbekämpfung" S. 170, Thieme Verlag Stuttgart 1977).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Weiterhin sind bestimmte substituierte Triazolinthione bekannt (vgl. DE-OS 27 07 801).

Über eins Wirksamkeit der vorbekannten Triazolinthione als Herbizide ist bisher nichts bekannt.

Ferner ist bekannt, daß auch substituierte Tetrazolinone herbizide Eigenschaften aufweisen. (EP-A-146 279).

Es wurden neue substituierte Triazolinone der allgemeinen Formel (I),

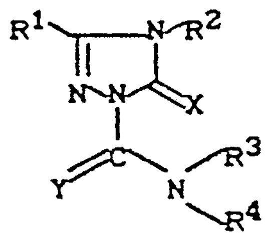

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils in Frage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils in Frage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^3$ und $R^4$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil stehen, wobei als Substituenten jeweils in Frage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach verknüpftes Alkandiyl bzw. Alkendiyl mit jeweils bis zu 4 Kohlenstoffatomen; außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aralkyloxy, Aryloxy, Aroyl oder Aryl mit jeweils 6 bis 10 Kohlenstoffatomen im Arylteil und gegebenenfalls 1 bis 6 Kohlenstoffatomen im Alkylteil stehen, wobei als Arylsubstituenten jeweils in Frage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen und Phenoxy und wobei als Alkylsubstituenten gegebenenfalls in Frage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel, enthalten kann, wobei als Substituenten in Frage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

X für Sauerstoff oder Schwefel steht und

Y für Sauerstoff oder Schwefel steht,

wobei jedoch X nur dann für Schwefel steht, wenn mindestens einer der Reste $R^3$ oder $R^4$ nicht gleichzeitig für Wasserstoff, Methyl oder Ethyl steht,
gefunden.

Die Verbindungen der Formel (I) können gegebenenfalls in Abhängigkeit von der Art der Substituenten $R^1$, $R^2$, $R^3$ und $R^4$ als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I),

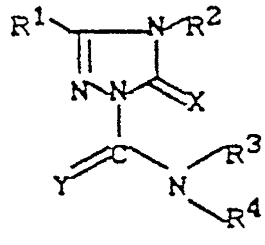

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils in Frage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils in Frage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^3$ und $R^4$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil stehen, wobei als Substituenten jeweils in Frage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach

verknüpftes Alkandiyl bzw. Alkendiyl mit jeweils bis zu 4 Kohlenstoffatomen; außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aralkyloxy, Aryloxy, Aroyl oder Aryl mit jeweils 6 bis 10 Kohlenstoffatomen im Arylteil und gegebenenfalls 1 bis 6 Kohlenstoffatomen im Alkylteil stehen, wobei als Arylsubstituenten jeweils in Frage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halcgenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen und Phenoxy und wobei als Alkylsubstituenten gegebenenfalls in Frage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel, enthalten kann, wobei als Substituenten in Frage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

X für Sauerstoff oder Schwefel steht und

Y für Sauerstoff oder Schwefel steht,

wobei jedoch X nur dann für Schwefel steht, wenn mindestens einer der Reste $R^3$ oder $R^4$ nicht gleichzeitig für Wasserstoff, Methyl oder Ethyl steht,

erhält, wenn man

a) 1-Chlor-(thio)carbonyltriazolinone der Formel (II),

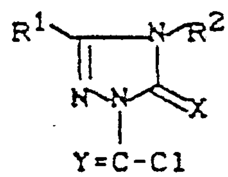

(II)

in welcher

$R^1$, $R^2$, X und Y die oben angegebene Bedeutung haben,

mit Aminen der Formel (III),

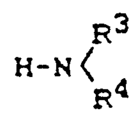

(III)

in welcher

$R^3$ und $R^4$ die oben angegebene Bedeutung haben,

gegebenenfalls in Gegenwart eines Verdünnungsmittels und gegebenenfalls in Gegenwart eines Säurebindemittels umsetzt, oder

b) für den Fall, daß $R^3$ Wasserstoff bedeutet, wenn man in 1-Stellung unsubstituierte Triazolinone der

Formel (IV),

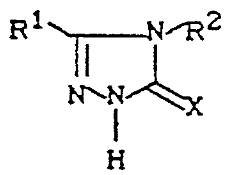

(IV)

in welcher

$R^1$, $R^2$ und X die oben angegebene Bedeutung haben,
mit Iso(thio)cyanaten der Formel (V),

$R^4$-N=C=Y (V)

in welcher

$R^4$ und Y die oben angegebene Bedeutung haben,
gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der allgemeinen Formel (I) eine erheblich höhere herbizide Potenz gegenüber Problemunkräutern als die aus dem Stand der Technik bekannten Stickstoffheterocyclen, wie beispielsweise das Imidazolin-2-on-1-carbonsäureisobutylamid, welche chemisch und wirkungsmäßig naheliegende Verbindungen sind.

Die erfindungsgemäßen substituierten Triazolinone sind durch die Formel (I) allgemein definiert. Bevorzugt sind Verbindungen der Formel (I), bei welchen

$R^1$ für Wasserstoff, Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, Ethoxymethyl, Methoxyethyl, Ethoxyethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ für Methyl, Ethyl, n- oder i-Propyl, n-, i-, s-oder t-Butyl, n- oder i-Pentyl, n- oder-i-Hexyl, für Allyl, Propargyl, Methoxy, Ethoxy, Methoxymethyl, Ethoxymethyl, Methoxyethyl, Ethoxyethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^3$ und $R^4$ unabhängig voneinander jeweils für Wasserstoff, Methyl, Ethyl, n- oder i-Propyl, n-, i-, s- oder t-Butyl, n- oder i-Pentyl, n -oder i-Hexyl, n- oder i-Heptyl, n- oder i-Octyl, n- oder i-Nonyl, n- oder i-Decyl, n- oder i-Dodecyl, für Allyl, Propenyl, n-oder i-Butenyl, n- oder i-Pentenyl, n- oder i-Hexenyl, Propargyl, n-oder i-Butinyl, n- oder i-Pentinyl, n- oder i-Hexinyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 4 Kohlenstoffatomen

im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl stehen, wobei als Substituenten im cyclischen und gegebenenfalls im aliphatischen Teil jeweils in Frage kommen: Fluor, Chlor, Brom, Methyl, Ethyl, n-oder i-Propyl, n-, i-, s- oder t-Butyl, Cyano, Methandiyl, Ethandiyl, Butandiyl oder Butadiendiyl; außerdem für im Heterocyclylteil gegebenenfalls ein- bis dreifach, gleich oder verschieden substituiertes Heterocyclylmethyl, Heterocyclylethyl oder Heterocyclylpropyl stehen, wobei als Heterocyclen jeweils in Frage kommen:

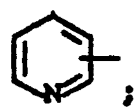 ; 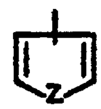 ; 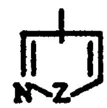 ; 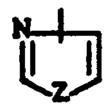 ;

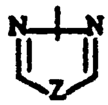 ; 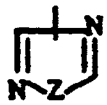 ; 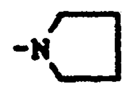 ; 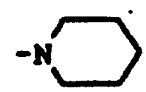 ;

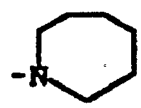 ; 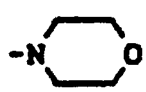 oder 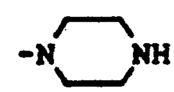

wobei Z jeweils für Sauerstoff oder Schwefel steht und wobei als Substituenten in Frage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl stehen, wobei als Phenylsubstituenten jeweils in Frage kommen:
Fluor, Chlor, Brom, Hydroxy, Cyano, Nitro, Methyl, Ethyl, n- oder i-Propyl, n-, i-, s-oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl und Phenoxy oder

$R^3$ und $R^4$ gemeinsam mit dem Stickstoffatom, an welches sie gebunden sind, für einen gegebenenfalls ein- bis dreifach, gleich oder verschieden substituierten Heterocyclus der Formel

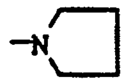 ; 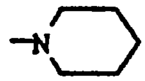 ; 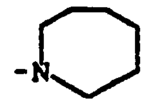 ; 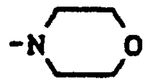 ;

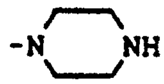 ; 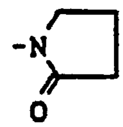 ; 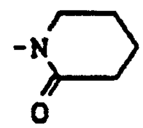 ; 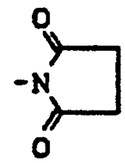 ;

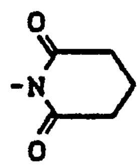 ; 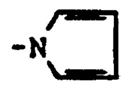 oder 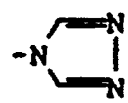 stehen,

wobei als Substituenten in Frage kommen: Methyl, Ethyl, n- oder i-Propyl, Chlor oder Trifluormethyl,

X für Sauerstoff oder Schwefel steht und

Y für Sauerstoff oder Schwefel steht, wobei

jedoch X nur dann für Schwefel steht, wenn mindestens einer der Reste $R^3$ oder $R^4$ nicht gleichzeitig für Wasserstoff, Methyl oder Ethyl steht.

Im einzelnen seien außer den bei den Herstellungsbeispielen genannten Verbindungen die folgenden substituierten Triazolinone der allgemeinen Formel (I) genannt:

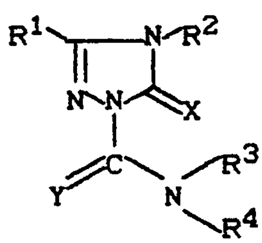 (I)

Tabelle 1

| $R^1$ | $R^2$ | $R^3$ | $R^4$ | X | Y |
|---|---|---|---|---|---|
| $CH_3$ | $C_2H_5$ | H | $-CH(CH_3)_2$ | O | O |
| $CH_3$ | $C_2H_5$ | H | $-CH(CH_3)_2$ | O | S |
| $CH_3$ | $CH_3$ | H | $-C(CH_3)_3$ | O | S |
| $CH_3$ | $CH_3$ | H | $-C(CH_3)_3$ | S | S |
| $CH_3$ | $C_2H_5$ | H | $-CH(CH_3)_2$ | S | O |
| $CH_3$ | $CH_3$ | H | 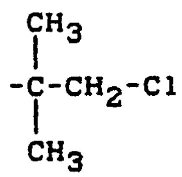 | S | O |
| $C_2H_5$ | $CH_3$ | H | $-C(CH_3)_3$ | S | O |

Tabelle 1 - Fortsetzung

| $R^1$ | $R^2$ | $R^3$ | $R^4$ | X | Y |
|---|---|---|---|---|---|
| $C_2H_5$ | $CH_3$ | H | $-C(CH_3)_3$ | O | S |
| $C_2H_5$ | $CH_3$ | H | 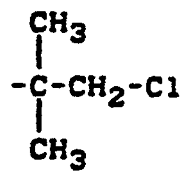 | O | S |
| $C_2H_5$ | $CH_3$ | H | 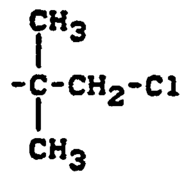 | S | O |
| $C_2H_5$ | $CH_3$ | H | 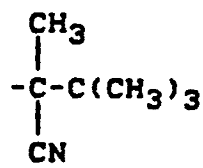 | S | O |
| $C_2H_5$ | $CH_3$ | H | 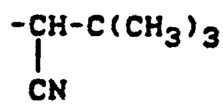 | S | O |
| $C_2H_5$ | $CH_3$ | H | 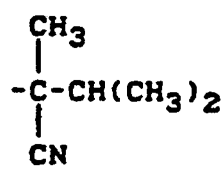 | O | O |
| $C_2H_5$ | $CH_3$ | H | 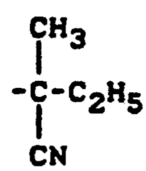 | S | O |
| $C_2H_5$ | $CH_3$ | H | 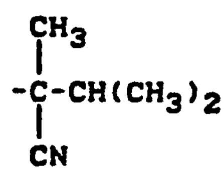 | S | O |

**Tabelle 1** - Fortsetzung

| $R^1$ | $R^2$ | $R^3$ | $R^4$ | X | Y |
|---|---|---|---|---|---|
| $C_2H_5$ | $CH_3$ | H | 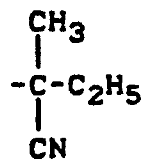 | O | S |
| $C_2H_5$ | $CH_3$ | H | 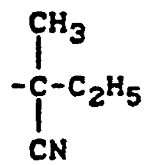 | S | S |
| $CH_3$ | $CH_3$ | H | 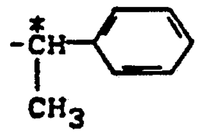 (-) | O | S |
| $CH_3$ | $CH_3$ | H | 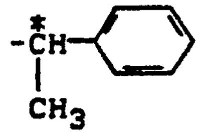 (-) | S | O |
| $C_2H_5$ | $CH_3$ | H | 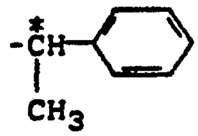 (-) | O | S |
| $C_2H_5$ | $CH_3$ | H | 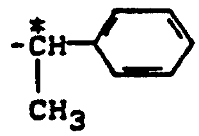 (-) | S | O |
| $C_2H_5$ | $CH_3$ | H | 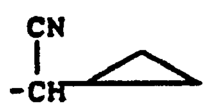 | S | O |
| $C_2H_5$ | $CH_3$ | H | 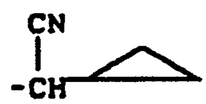 | O | S |
| $C_2H_5$ | $CH_3$ | H | 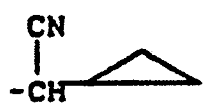 | S | S |

Tabelle 1 - Fortsetzung

| $R^1$ | $R^2$ | $R^3$ | $R^4$ | X | Y |
|---|---|---|---|---|---|
| $C_2H_5$ | $CH_3$ | H | 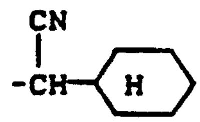 | O | S |
| $C_2H_5$ | $CH_3$ | H | 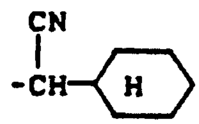 | S | O |
| $(CH_3)_2CH-$ | $CH_3$ | H | $-C(CH_3)_3$ | S | O |
| $(CH_3)_2CH-$ | $CH_3$ | H | 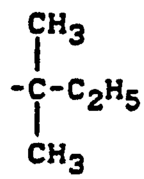 | S | O |
| $(CH_3)_2CH-$ | $CH_3$ | H | 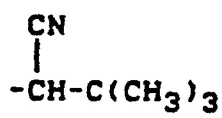 | S | O |
| $(CH_3)_2CH-$ | $CH_3$ | H | 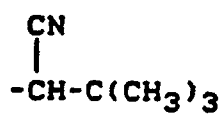 | O | S |
| $(CH_3)_2CH-$ | $CH_3$ | H | 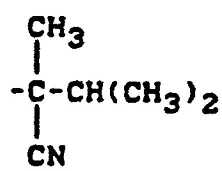 | S | O |
| $(CH_3)_2CH-$ | $CH_3$ | H | 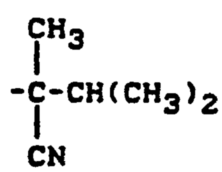 | O | S |
| $(CH_3)_2CH-$ | $CH_3$ | H | 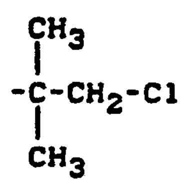 | O | S |

<u>Tabelle 1</u> - Fortsetzung

| $R^1$ | $R^2$ | $R^3$ | $R^4$ | X | Y |
|---|---|---|---|---|---|
| $(CH_3)_2CH-$ | $CH_3$ | H | 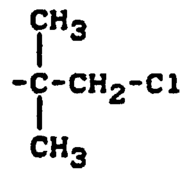 | S | O |
| $(CH_3)_2CH-$ | $CH_3$ | H | 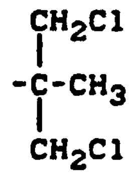 | S | O |
| $(CH_3)_2CH-$ | $CH_3$ | H | 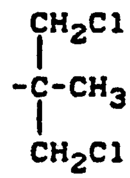 | O | S |
| $CH_3$ | $CH_3$ | H | 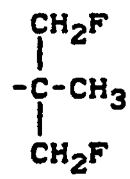 | S | O |
| $CH_3$ | $CH_3$ | H | 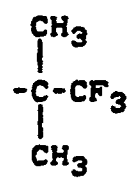 | S | O |
| $C_2H_5$ | $CH_3$ | H | 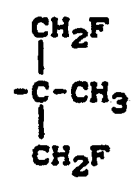 | S | O |
| $C_2H_5$ | $CH_3$ | H | 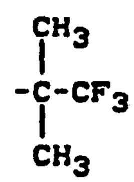 | S | O |

Tabelle 1 - Fortsetzung

| $R^1$ | $R^2$ | $R^3$ | $R^4$ | X | Y |
|---|---|---|---|---|---|
| $C_2H_5$ | $CH_3$ | H | 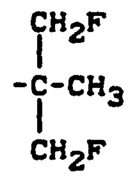 | O | S |
| $C_2H_5$ | $C_2H_5$ | H | $-C(CH_3)_3$ | O | O |
| $C_2H_5$ | $C_2H_5$ | H | 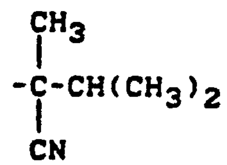 | O | O |
| $C_2H_5$ | $C_2H_5$ | H | $-C(CH_3)_3$ | S | O |
| $C_2H_5$ | $C_2H_5$ | H | $-C(CH_3)_3$ | O | S |

Verwendet man beispielsweise 1-Chlorcarbonyl-3-ethoxymethyl-4-methyl-1,2,4-triazolin-5-on und Allylamin als Ausgangsstoffe, so läßt sich der Reaktionsablauf des erfindungsgemäßen Verfahrens (a) durch das folgende Formelschema darstellen:

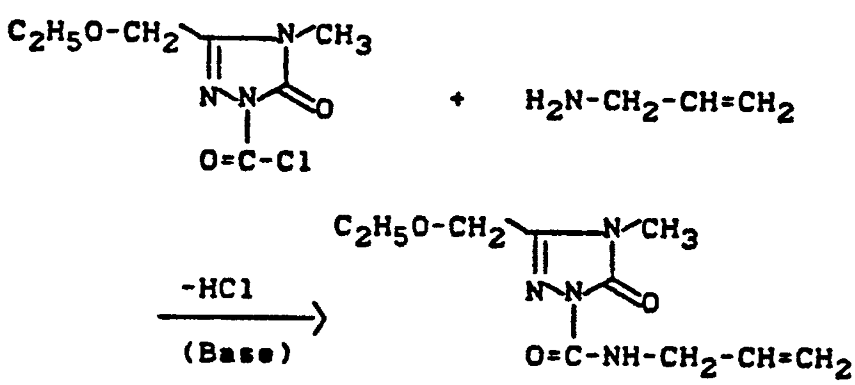


Verwendet man beispielsweise 3,4-Dimethyl-1H-1,2,4-triazolin-5-on und Isopropylisocyanat als Ausgangsstoffe, so läßt sich der Reaktionsablauf des erfindungsgemäßen Verfahrens (b) durch das folgende Formelschema darstellen:

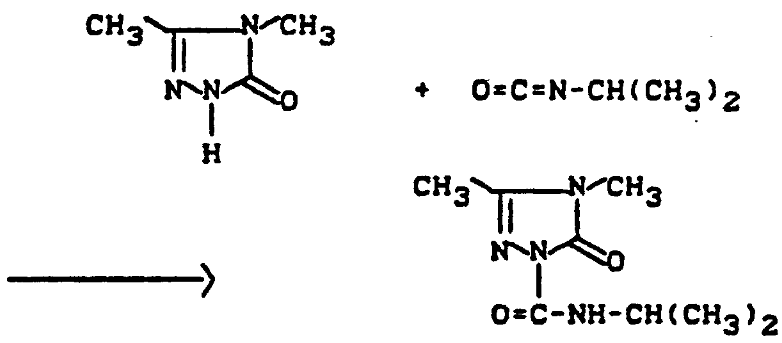

Die zur Durchführung des erfindungsgemäßen Verfahrens (a) als Ausgangsstoffe benötigten Chlor(thio)-carbonyltriazolinone sind durch die Formel (II) allgemein definiert. In dieser Formel (II) stehen $R^1$, $R^2$, X und Y vorzugsweise bzw. besonders bevorzugt für diejenigen Reste, die bereits im Zusammenhang mit der Beschreibung der erfindungsgemäßen Stoffe der Formel (I) als bevorzugt bzw. besonders bevorzugt für diese Substituenten genannt wurden.

Die Chlor(thio)carbonyltriazolinone der Formel (II) sind noch nicht bekannt.

Man erhält sie, wenn man in 1-Stellung unsubstituierte Triazolinone der Formel (IV),

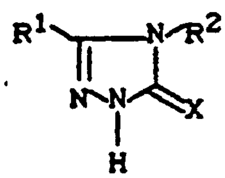 (IV)

in welcher

$R^1$, $R^2$ und X die oben angegebene Bedeutung haben,

mit (Thio)Phosgen der Formel (VI),

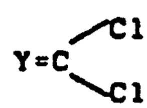 (VI)

in welcher

Y die oben angegebene Bedeutung hat,

gegebenenfalls in Gegenwart eines Verdünnungsmittels wie beispielsweise Toluol oder Acetonitril und gegebenenfalls in Gegenwart eines Säurebindemittels wie beispielsweise Triethylamin bei Temperaturen zwischen +20 °C und +150 °C umsetzt.

Die zur Durchführung des erfindungsgemäßen Verfahrens (a) weiterhin als Ausgangsstoffe benötigten Amine sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten in 1-Stellung unsubstituierten Triazolinone sind durch die Formel (IV) allgemein definiert. In dieser Formel (IV) stehen $R^1$, $R^2$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die in 1-Stellung unsubstituierten Triazolinone der Formel (IV) sind teilweise bekannt (vgl. z. B. Chem. Ber. 98, 3034 [1965]; Bull. Soc. Chim, Fr. 1975, 1191; Bull. Soc. Chim. Fr. 1962, 1365; DE-OS 23 36 827). Man erhält die bekannten, ebenso wie die nicht bekannten Verbindungen der Formel (IV) in Analogie zu

bekannten Verfahren (vgl. z. B. Arch. Pharm. 301, 827 [1968]; J. Heterocycl. Chem. 15, 237 [1978]; Tetrahedron 32, 2347 [1976]; Roczn. Chem. 42, 247 [1968] sowie die Herstellungsbeispiele).

Die zur Durchführung des erfindungsgemäßen Verfahrens (b) weiterhin als Ausgangsstoffe benötigten Iso(thio)cyanate sind durch die Formel (V) allgemein definiert. In dieser Formel (V) stehen $R^4$ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate sind allgemein bekannte Verbindungen der organischen Chemie (vgl. z. B. Saul Patai, "The Chemistry of Cyanates and their Thioderivatives" J. Wiley & Sons, New York 1977).

Als Verdünnungsmittel zur Durchführung des erfindungsgemäßen Verfahrens (a) kommen vorzugsweise inerte organische Lösungsmittel infrage. Hierzu gehören insbesondere aliphatische, alicyclische oder aromatische, gegebenenfalls halogenierte Kohlenwasserstoffe, wie beispielsweise Benzin, Ligroin, Benzol, Toluol, Xylol, Chlorbenzol, Petrolether, Pentan, Hexan, Heptan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Basen wie Pyridin.

Das erfindungsgemäße Verfahren (a) wird gegebenenfalls in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Es ist auch möglich das als Reaktionspartner verwendete Amin der Formel (III) in entsprechendem Überschuß gleichzeitig als Säurebindemittel einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und +150 °C, vorzugsweise bei Temperaturen zwischen +10 °C und +80 °C.

Das erfindungsgemäße Verfahren (a) wird üblicherweise unter Normaldruck durchgeführt. Es ist jedoch auch möglich unter erhöhtem Druck zu arbeiten.

Zur Durchführung des erfindungsgemäßen Verfahrens (a) setzt man pro Mol an 1-Chlor-(thio)carbonyltriazolinon der Formel (II) im allgemeinen 1,0 bis 5,0 Mol, vorzugsweise 1,0 bis 2,5 Mol am Amin der Formel (III) und gegebenenfalls 1,0 bis 2,5 Mol an Säurebindemittel ein. Die Reaktionsdurchführung, Aufarbeitung und Isolierung der Reaktionsprodukte erfolgt in Analogie zu allgemein bekannten Verfahren.

Als Verdünnungmittel zur Durchführung des erfindungsgemäßen Verfahrens (b) kommen ebenfalls inerte organische Lösungsmittel in Frage. Vorzugsweise verwendet man die bei Verfahren (a) genannten Verdünnungsmittel.

Das erfindungsgemäße Verfahren (b) kann gegebenenfalls in Gegenwart eines basischen Reaktionshilfsmittels durchgeführt werden. Als solche kommen alle üblichen anorganischen und organischen Basen infrage. Vorzugsweise verwendet man tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Der Zusatz solcher Katalysatoren ist jedoch nicht zwingend erforderlich.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und +150 °C, vorzugsweise bei Temperaturen zwischen +40 °C und +120 °C.

Das erfindungsgemäße Verfahren (b) wird üblicherweise unter Normaldruck durchgeführt. Es ist jedoch auch möglich, insbesondere bei gasförmigen Ausgangsverbindungen, unter erhöhtem Druck zu arbeiten.

Zur Durchführung des erfindungsgemäßen Verfahrens (b) setzt man pro Mol an in 1-Stellung unsubstituiertem Triazolinon der Formel (IV) im allgemeinen 1,0 bis 5,0 Mol, vorzugsweise 1,0 bis 2,5 Mol an Iso(thio)cyanat der Formel (V) und gegebenenfalls 1,0 bis 2,5 Mol an Reaktionshilfsmittel ein. Die Reaktionsdurchführung, Aufarbeitung und Isolierung der Reaktionsprodukte erfolgt in Analogie zu allgemein bekannten Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung dikotyler Unkräuter insbesondere in monokotylen Kulturen, wie beispielsweise Mais einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser. Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; 2-[4-(2,4-Dichlorphenoxy)-phenoxy]-propionsäure, deren Methyl- oder deren Ethylester; 2-{4-[(6-Chlor-2-benzoxazolyl)-oxy]-phenoxy}-propansäure, deren Methyl- oder deren Ethylester; 2-[4-(3,5-Dichlorpyrid-2-yloxy)-phenoxy]-propionsäure-(trimethylsilylmethylester); 3-(Ethoxycarbonylaminophenyl)-N-(3'-methylphenyl)-carbamat; N-(1-Ethylpropyl)-3,4-dimethyl-2,6-dinitroanilin; Methyl-5-(2,4-dichlorphenoxy)-2-nitrobenzoat; N,N-Dimethyl-N'-(4-isopropylphenyl)-harnstoff; Methyl-6,6-dimethyl-2,4-dioxo-3-[1-(2-propenyloxyamino)-butyliden]-cyclohexancarbonsäure; 2-[1-(Ethoximino)-butyl]-3-hydroxy-5-[tetrahydro-(2H)-thiopyran-3-yl]-2-cyclohexen-1-on; 2-[1-(Ethoxamino)-butyliden]-5-(2-ethylthiopropyl)-1,3-cyclohexadion;Methyl-2-[4,5-dihydro-4-methyl-4-(1-methylethyl)-5-oxo-1H-imidazol-2-yl]-4(5)-methylbenzoat; 2-[5-Methyl-5-(1-methylethyl)-4-oxo-2-imidazolin-2-yl]-3-chinolincarbonsäure; 3,5-Dibrom-4-hydroxy-benzonitril; 3,5-Diiod-4-hydroxybenzonitril; N-Methyl-2-(1,3-benzthiazol-2-yloxy)-acetanilid; 3-[[[[(4-Methoxy-6-methyl-1,3,5-triazin-2-yl)-amino]-carbonyl]-amino]-sulfonyl]-thiophen-2-carbonsäure-methylester; 2-Chlor-4-ethylamino-6-isopropylamino-1,3,5-triazin; 2-Chlor-4-ethylamino-6-(3-cyanopropylamino)-1,3,5-triazin; 3-Isopropyl-2,1,3-benzothiadiazin-4-on-2,2-dioxid; 0-(6-Chlor-3-phenyl-pyridazin-4-yl)-S-octyl-thiocarbonat oder 4-(2,4-Dichlorphenoxy)buttersäure sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

## Herstellungsbeispiele

### Beispiel 1

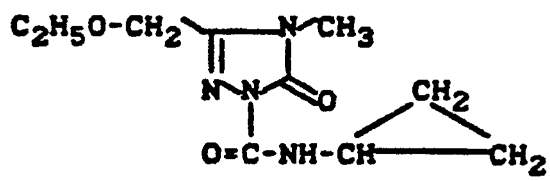

(Verfahren a)

Zu 4,4 g (0,02 Mol) 1-Chlorcarbonyl-3-ethoxymethyl-4-methyl-1,2,4-triazolin-5-on in 80 ml Dichlormethan tropft man unter Rühren 2,3 g (0,4 Mol) Cyclopropylamin so, daß die Reaktionstemperatur 40 °C nicht übersteigt. Nach beendeter Zugabe rührt man vier Stunden bei Raumtemperatur, filtriert dann ausgefallenes Cyclopropylaminhydrochlorid ab, engt das Filtrat im Vakuum ein, nimmt den öligen Rückstand in 150 ml Dichlormethan auf, wäscht dreimal mit jeweils 50 ml Wasser, trocknet über Natriumsulfat und entfernt das Lösungsmittel im Vakuum.

Man erhält 4,2 g (88 % der Theorie) an 1-Cyclopropylaminocarbonyl-3-ethoxymethyl-4-methyl-1,2,4-triazolin-5-on als Öl; $^1$H-NMR ($CDCl_3$/TMS) $\delta$ = 2.85 ppm (m; 1H).

### Herstellung der Ausgangsverbindungen

Beispiel II-1

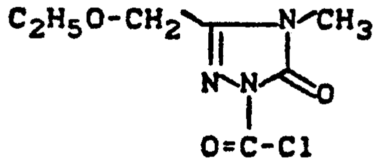

15,7 g (0,1 Mol) 3-Ethoxymethyl-4-methyl-1H-1,2,4-triazolin-5-on in 150 ml Acetonitril werden unter Einleiten von Phosgen auf 80 °C erwärmt. Insgesamt leitet man 20 g (0,2 Mol) Phosgen ein. Ab 60 °C findet eine lebhafte Chlorwasserstoffentwicklung statt. Nach beendeter Phosgen-Einleitung rührt man weitere 5 Stunden bei 80 °C, entfernt überschüssiges Phosgen und Chlorwasserstoff durch Ausblasen mit Stickstoff und filtriert die Mischung bei 20 °C. Das Filtrat wird mit 1 l Cyclohexan verrührt, das ausgefallene Produkt abgesaugt, mit Cyclohexan gewaschen und getrocknet.

Man erhält 18,2 g (82 % der Theorie) an 1-Chlorcarbonyl-3-ethoxymethyl-4-methyl-1,2,4-triazolin-5-on vom Schmelzpunkt Fp. 107 °C.

Beispiel IV-1

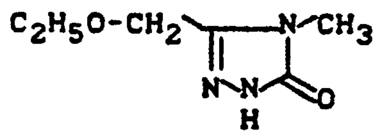

Zu 23,4 g (0,26 Mol) 4-Methylsemicarbazid in 500 ml Dichlormethan gibt man unter Rühren und Eiskühlung 50 g (0,23 Mol) Ethoxyessigsäureanhydrid, rührt nach beendeter Zugabe 15 Stunden bei Raumtemperatur und engt dann im Vakuum ein. Das so erhältliche Rohprodukt wird in 800 ml Wasser mit 15 g Natriumhydroxid versetzt, 3 Stunden bei 100 °C gerührt, mit verdünnter Salzsäure neutralisiert und im Vakuum eingeengt. Der Rückstand wird in 700 ml Essigester/Ethanol (1 : 1) aufgenommen, filtriert, das Filtrat wieder eingeengt und das verbleibende Öl durch Verreiben mit Ether kristallisiert.

Man erhält 24 g (59 % der Theorie) an 3-Ethoxymethyl-4-methyl-1,2,4-(1H)-triazolin-5-on vom Schmelzpunkt 92 °C.

Beispiel 2

(Verfahren b)

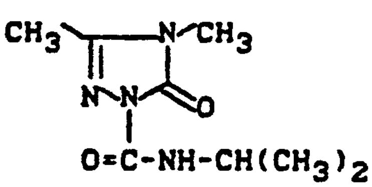

2,2 g (0,02 Mol) 3,4-Dimethyl-1H-1,2,4-triazolin-5-on (vgl. Bull. Soc. Chim. Fr. 1975, 1191) in 100 ml Toluol werden mit 4,25 g (0,05 Mol) Isopropylisocyanat versetzt und 2 Stunden bei 120 °C gerührt. Das erkaltete Reaktionsgemisch wird filtriert und das Filtrat im Vakuum eingeengt.

Man erhält 2,4 g (60 % der Theorie) an 1-Isopropylaminocarbonyl-3,4-dimethyl-1,2,4-triazolin-5-on vom Schmelzpunkt 81 °C.

In entsprechender Weise und gemäß den allgemeinen Angaben zur Herstellung erhält man die folgenden substituierten Triazolinone der allgemeinen Formel (I):

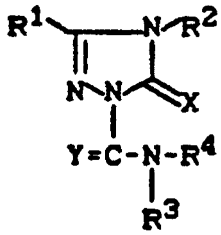

(I)

Tabelle 2

| Bsp.-Nr. | $R^1$ | $R^2$ | 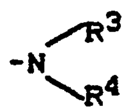 | X | Y | physikal. Eigenschaften |
|---|---|---|---|---|---|---|
| 3 | $CH_3$ | $CH_3$ | 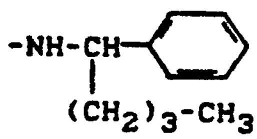 | O | O | $^1H$-NMR*): 5.0 (m) |
| 4 | $CH_3$ | $CH_3$ | 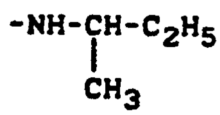 | O | O | $^1H$-NMR*): 4.0 (m) |
| 5 | $CH_3$ | $CH_3$ | $-NH-CH_2-CH(CH_3)_2$ | O | O | Fp. 64 °C |
| 6 | $CH_3$ | $CH_3$ | $-NH-(CH_2)_5-CH_3$ | O | O | Fp. 41 °C |
| 7 | $CH_3$ | $CH_3$ | $-NH-(CH_2)_3-OC_2H_5$ | O | O | Fp. 70 °C |
| 8 | $CH_3$ | $CH_3$ | $-NH-CH_2-C(CH_3)_3$ | O | O | Fp.63-66° C |
| 9 | $CH_3$ | $CH_3$ | 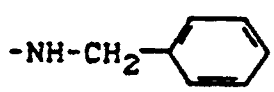 | O | O | Fp. 178° C |
| 10 | $C_2H_5$ | $CH_3$ | $-NH-CH(CH_3)_2$ | O | O | Fp. 93 °C |
| 11 | $CH_3$ | $CH_3$ | 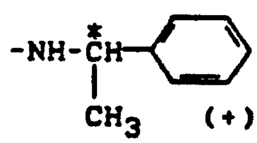 (+) | O | O | Fp. 110° C |
| 12 | $CH_3$ | $CH_3$ | 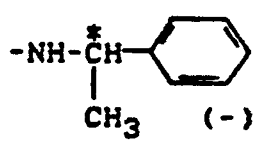 (-) | O | O | Fp. 98 °C |
| 13 | $C_2H_5$ | $CH_3$ | 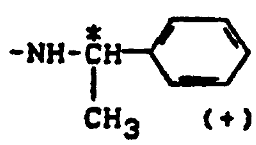 (+) | O | O | $^1H$-NMR*): 5.2 (m) |

Tabelle 2 - Fortsetzung

| Bsp.-Nr. | $R^1$ | $R^2$ | 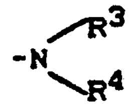 | X | Y | physikal. Eigenschaften |
|---|---|---|---|---|---|---|
| 14 | $C_2H_5$ | $CH_3$ | 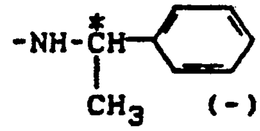 | O | O | $^1H$-NMR*): 5.2 (m) |
| 15 | $C_2H_5O-CH_2-$ | $CH_3$ | $-NH-CH(CH_3)_2$ | O | O | Fp. 80 °C |
| 16 | $C_2H_5O-CH_2-$ | $CH_3$ | $-NH-C(CH_3)_3$ | O | O | $^1H$-NMR*): 1.4 (s) |
| 17 | $CH_3$ | $CH_3$ | $-NH-C(CH_3)_3$ | O | O | Fp.112-114° C |
| 18 | $CH_3$ | $CH_3$ | $-NH-(CH_2)_2-OCH_3$ | O | O | $^1H$-NMR*): 3.4 (s) |
| 19 | $CH_3$ | $CH_3$ | $-NH-(CH_2)_2-CH_3$ | O | O | Fp. 58 °C |
| 20 | $CH_3$ | $CH_3$ | 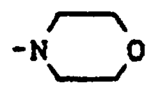 | O | O | $^1H$-NMR*): 3.6 (m) |
| 21 | $CH_3$ | $CH_3$ | 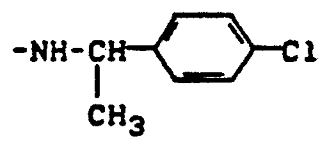 | O | O | $^1H$-NMR*): |
| 22 | $CH_3$ | $CH_3$ | 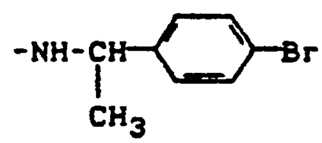 | O | O | Fp. 84 °C |
| 23 | $CH_3$ | $CH_3$ | 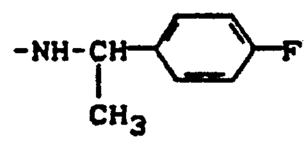 | O | O | Fp. 78 °C |
| 24 | $C_2H_5$ | $CH_3$ | 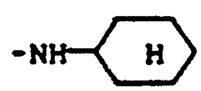 | O | O | Fp. 103° C |

<u>Tabelle 2</u> - Fortsetzung

| Bsp.-Nr. | $R^1$ | $R^2$ | $-N(R^3)(R^4)$ | X | Y | physikal. Eigenschaften |
|---|---|---|---|---|---|---|
| 25 | $C_2H_5$ | $CH_3$ | $-NH-CH(CH_3)-C_2H_5$ | O | O | Fp. 74 °C |
| 26 | $C_2H_5$ | $CH_3$ | $-NH-CH_2-CH(CH_3)_2$ | O | O | Fp. 67 °C |
| 27 | $C_2H_5$ | $CH_3$ | $-NH-C(CH_3)_3$ | O | O | Fp. 126° C |
| 28 | $C_2H_5$ | $CH_3$ | $-NH-(CH_2)_5-CH_3$ | O | O | Fp. 37 °C |
| 29 | $C_2H_5$ | $CH_3$ | $-NH-(CH_2)_2-CH_3$ | O | O | Fp. 65 °C |
| 30 | $C_2H_5$ | $CH_3$ | $-NH-CH_2-C(CH_3)_3$ | O | O | Fp. 70 °C |
| 31 | $CH_3$ | $CH_3$ | $-NH-CH_2-$ 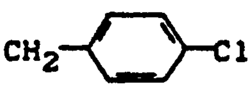 | O | O | Fp. 96 °C |
| 32 | $C_2H_5$ | $CH_3$ | -NH 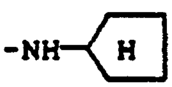 | O | O | Fp. 94 °C |
| 33 | $C_2H_5$ | $CH_3$ | $-NH-(CH_2)_2-CH(CH_3)_2$ | O | O | $^1H$-NMR*): 1.5 (m) |
| 34 | $C_2H_5$ | $CH_3$ | $-NH-CH_2-CH(CH_3)-C_2H_5$ | O | O | Fp. 49 °C |
| 35 | $C_2H_5$ | $CH_3$ | $-NH-(CH_2)_3-N(CH_3)_2$ | O | O | $^1H$-NMR*): 2.2 (s) |
| 36 | $C_2H_5$ | $CH_3$ | $-NH-(CH_2)_4-CH_3$ | O | O | $^1H$-NMR*): 3.4 (q) |
| 37 | $C_2H_5$ | $CH_3$ | -NH 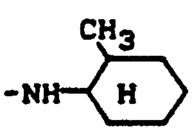 | O | O | Fp. 74 °C |

__Tabelle 2__ - Fortsetzung

| Bsp.-Nr. | $R^1$ | $R^2$ | 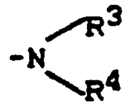 | X | Y | physikal. Eigenschaften |
|---|---|---|---|---|---|---|
| 38 | $C_2H_5$ | $CH_3$ | 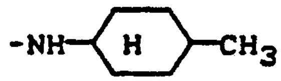 | O | O | Fp. 94 °C |
| 39 | $C_2H_5$ | $CH_3$ | $-NH-CH_2-CH_2-Cl$ | O | O | Fp. 98 °C |
| 40 | $C_2H_5$ | $CH_3$ | 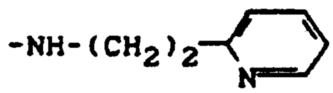 | O | O | Fp. 61 °C |
| 41 | $C_2H_5$ | $CH_3$ | 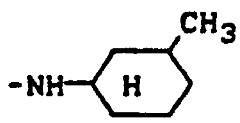 | O | O | Fp. 102°C |
| 42 | $C_2H_5$ | $CH_3$ | 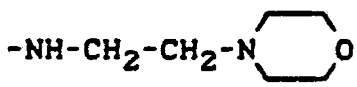 | O | O | $^1$H-NMR*): 3.7 (m) |
| 43 | H | $CH_3$ | $-NH-CH(CH_3)_2$ | O | O | Fp. 167°C |
| 44 | H | $CH_3$ | 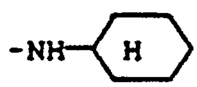 | O | O | Fp. 168°C |
| 45 | H | $CH_3$ | 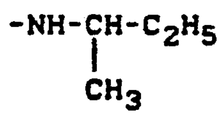 | O | O | Fp. 118°C |
| 46 | H | $CH_3$ | $-NH-CH_2-CH(CH_3)_2$ | O | O | Fp. 167°C |
| 47 | H | $CH_3$ | $-NH-C(CH_3)_3$ | O | O | Fp. 147°C |
| 48 | H | $CH_3$ | 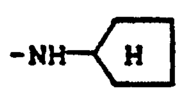 | O | O | Fp. 190°C |
| 49 | H | $CH_3$ | $-NH-(CH_2)_2-CH(CH_3)_2$ | O | O | Fp. 161°C |

Tabelle 2 - Fortsetzung

| Bsp.-Nr. | $R^1$ | $R^2$ | 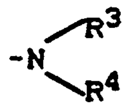 | X | Y | physikal. Eigenschaften |
|---|---|---|---|---|---|---|
| 50 | H | $CH_3$ | 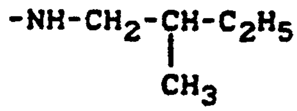 | O | O | Fp. 134° C |
| 51 | H | $CH_3$ | 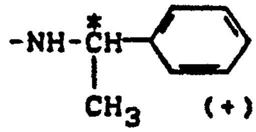 (+) | O | O | Fp. 177° C |
| 52 | H | $CH_3$ | 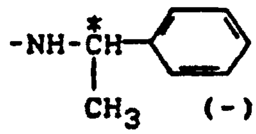 (-) | O | O | Fp. 202° C |
| 53 | $(CH_3)_2CH-$ | $CH_3$ | $-NH-CH(CH_3)_2$ | O | O | Fp. 107° C |
| 54 | $C_2H_5O-CH_2-$ | $CH_3$ | 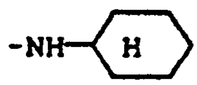 | O | O | $^1H$-NMR*): 3.8 (m) |
| 55 | $C_2H_5O-CH_2-$ | $CH_3$ | 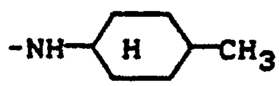 | O | O | $^1H$-NMR*): 3.8 (m) |
| 56 | $CH_3$ | $CH_3$ | 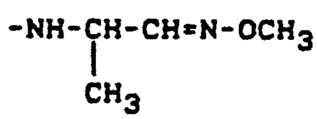 | O | O | $^1H$-NMR*): 4.7 (m) |
| 57 | $C_2H_5$ | $CH_3$ | 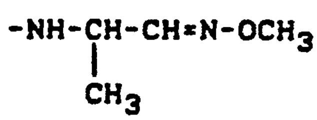 | O | O | $^1H$-NMR*): 4.7 (m) |
| 58 | H | $CH_3$ | 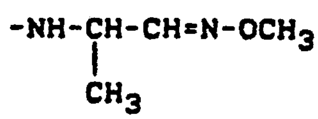 | O | O | $^1H$-NMR*): 4.7 (m) |
| 59 | $C_2H_5O-CH_2-$ | $CH_3$ | 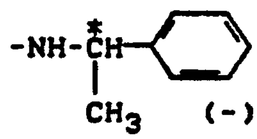 (-) | O | O | $^1H$-NMR*): 5.2 (m) |

<u>Tabelle 2</u> - Fortsetzung

| Bsp.-Nr. | $R^1$ | $R^2$ | 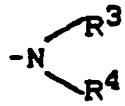 | X | Y | physikal. Eigen-schaften |
|---|---|---|---|---|---|---|
| 60 | $(CH_3)_2CH-$ | $CH_3$ | 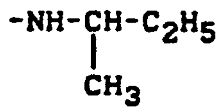 | O | O | Fp. 65 °C |
| 61 | $(CH_3)_2CH-$ | $CH_3$ | $-NH-C(CH_3)_3$ | O | O | Fp. 131° C |
| 62 | $(CH_3)_2)CH-$ | $CH_3$ | 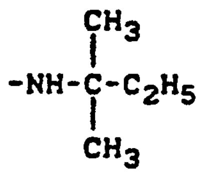 | O | O | $^1H$-NMR*): 0.9 (t) |
| 63 | $(CH_3)_2CH-$ | $CH_3$ | 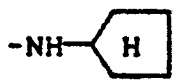 | O | O | Fp. 110° C |
| 64 | $(CH_3)_2CH-$ | $CH_3$ | 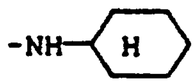 | O | O | Fp. 138° C |
| 65 | $(CH_3)_2CH-$ | $CH_3$ | 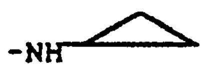 | O | O | Fp. 64 °C |
| 66 | $(CH_3)_2CH-$ | $CH_3$ | 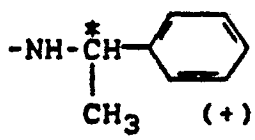 (+) | O | O | $^1H$-NMR*): 5.2 (m) |
| 67 | $(CH_3)_2CH-$ | $CH_3$ | 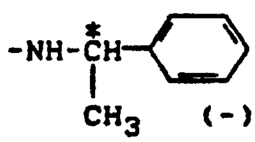 (-) | O | O | $^1H$-NMR*): 5.2 (m) |
| 68 | $CH_3$ | $CH_3$ | 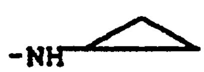 | O | O | Fp.147-150° C |
| 69 | $CH_3$ | $CH_3$ | 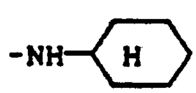 | O | O | Fp.121-123° C |

Tabelle 2 - Fortsetzung

| Bsp.-Nr. | $R^1$ | $R^2$ | 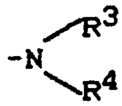 | X | Y | physikal. Eigenschaften |
|---|---|---|---|---|---|---|
| 70 | $CH_3$ | $CH_3$ | 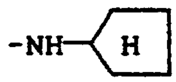 | O | O | Fp.91-92° C |
| 71 | $C_2H_5$ | $CH_3$ | 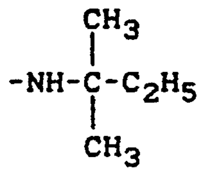 | O | O | Fp. 87 °C |
| 72 | $C_2H_5$ | $CH_3$ | 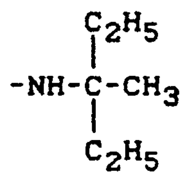 | O | O | Fp. 90 °C |
| 73 | $C_2H_5$ | $CH_3$ | 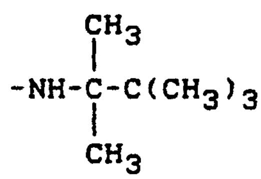 | O | O | Fp. 93 °C |
| 74 | $C_2H_5$ | $CH_3$ | 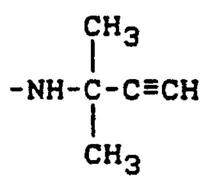 | O | O | Fp. 154° C |
| 75 | $C_2H_5$ | $CH_3$ | 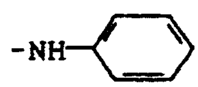 | O | O | Fp. 170° C |
| 76 | $C_2H_5$ | $CH_3$ | 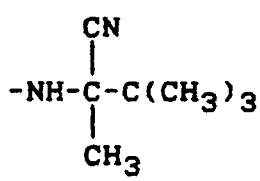 | O | O | |

**Tabelle 2 - Fortsetzung**

| Bsp.-Nr. | $R^1$ | $R^2$ | 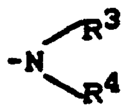 | X | Y | physikal. Eigenschaften |
|---|---|---|---|---|---|---|
| 77 | $C_2H_5$ | $CH_3$ | 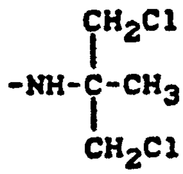 | O | O | Fp. 97 °C |
| 78 | $C_2H_5$ | $CH_3$ | 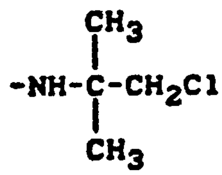 | O | O | Fp. 106° C |
| 79 | $C_2H_5$ | $CH_3$ | 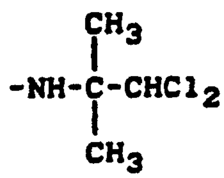 | O | O | |
| 80 | $C_2H_5$ | $CH_3$ | 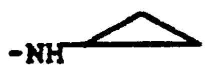 | O | O | $^1$H-NMR*): 2.8 (m) |
| 81 | $C_2H_5$ | $CH_3$ | 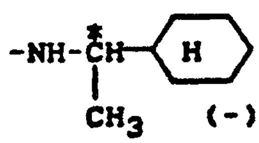 (-) | O | O | $^1$H-NMR*): 3.8 (m) |
| 82 | $C_2H_5$ | $CH_3$ | 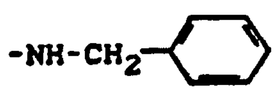 | O | O | Fp. 143° - 145° C |
| 83 | $C_2H_5$ | $CH_3$ | $-NH-(CH_2)_3-OC_2H_5$ | O | O | |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Anwendungsbeispiele

In dem folgenden Anwendungsbeispiel wurde die nachstehend aufgeführte Verbindung als Vergleichsubstanz eingesetzt:

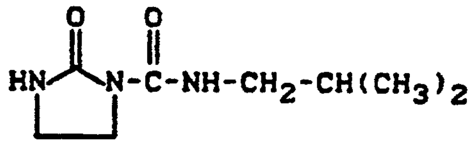 (A)

Imidazolidin-2-on-1-carbonsäureisobutylamid
(bekannt aus K. H. Büchel, "Pflanzenschutz und Schädlingsbekämpfung" S. 170, Thieme Verlag Stuttgart 1977).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung
Eine deutliche Überlegenheit in der Wirksamkeit bei vergleichbarer Nutzpflanzenselektivität gegen Unkräuter wie beispielsweise Abutilon, Datura und Galinsoga gegenüber der Vergleichssubstanz (A) zeigen in diesem Test z.B. die Verbindungen gemäß folgender Herstellungsbeispiele: (22) und (27).

**Patentansprüche**

1. Substituierte Triazolinone der Formel (I),

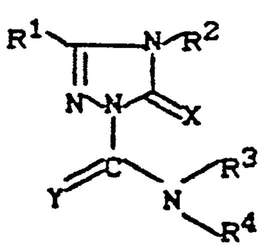 (I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils in Frage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils in Frage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^3$ und $R^4$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil stehen, wobei als Substituenten jeweils in Frage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach verknüpftes Alkandiyl bzw. Alkendiyl mit jeweils bis zu 4 Kohlenstoffatomen; außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; außerdem für jeweils geradkettiges oder verzweigtes Alkoxy, mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aralkyloxy, Aryloxy, Aroyl oder Aryl mit jeweils 6 bis 10 Kohlenstoffatomen im Arylteil und gegebenenfalls 1 bis 6 Kohlenstoffatomen im Alkylteil stehen, wobei als Arylsubstituenten jeweils in Frage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6

Kohlenstoffatomen und gegebenenfalls 1 bis 9 gleichen oder verschiedenen Halogenatomen, Cycloalkyl mit 3 bis 6 Kohlenstoffatomen und Phenoxy und wobei als Alkylsubstituenten gegebenenfalls in Frage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel, enthalten kann, wobei als Substituenten in Frage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo-oder Thionogruppen,

X für Sauerstoff oder Schwefel steht und

Y für Sauerstoff oder Schwefel steht,

wobei jedoch X nur dann für Schwefel steht, wenn mindestens einer der Reste $R^3$ oder $R^4$ nicht gleichzeitig für Wasserstoff, Methyl oder Ethyl steht.

**2.** Substituierte Triazolinone der Formel (I) gemäß Anspruch 1, in welcher

$R^1$ für Wasserstoff, Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, Ethoxymethyl, Methoxyethyl, Ethoxyethyl, für geradkettiges oder verzweigtes Halogenalkyl mit 1 bis 4 Kohlenstoffatomen und 1 bis 9 gleichen oder verschiedenen Halogenatomen, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten jeweils in Frage kommen: Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy oder Trifluormethylthio,

$R^2$ für Methyl, Ethyl, n- oder i-Propyl, n-, i-, s-oder t-Butyl, n- oder i-Pentyl, n- oder i-Hexyl, für Allyl, Propargyl, Methoxy, Ethoxy, Methoxymethyl, Ethoxymethyl, Methoxyethyl, Ethoxyethyl, für geradkettiges oder verzweigtes Halogenalkyl mit 1 bis 4 Kohlenstoffatomen und 1 bis 9 gleichen oder verschiedenen Halogenatomen, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^3$ und $R^4$ unabhängig voneinander jeweils für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, n- oder i-Nonyl, n- oder i-Decyl, n- oder i-Dodecyl, für Allyl, Propenyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 4 Kohlenstoffatomen und 1 bis 9 gleichen oder verschiedenen Halogenatomen, jeweils geradkettiges oder verzweigtes Halogenalkenyl oder Halogenalkinyl mit jeweils 3 bis 5 Kohlenstoffatomen und 1 bis 3 Halogenatomen, für jeweils geradkettiges oder verzweigtes Cyanalkyl mit 1 bis 4 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl stehen, wobei als Substituenten im cyclischen und gegebenenfalls im aliphatischen Teil jeweils in Frage kommen: Fluor, Chlor, Brom, Methyl, Ethyl, n-oder i-Propyl, n-, i-, s- oder t-Butyl, Cyano, Methandiyl, Ethandiyl, Butandiyl oder Butadiendiyl;

außerdem für im Heterocyclylteil gegebenenfalls ein- bis dreifach, gleich oder verschieden substituiertes Heterocyclylmethyl, Heterocyclylethyl oder Heterocyclylpropyl stehen, wobei als Heterocyclen jeweils in Frage kommen:

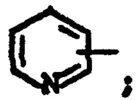 ; 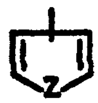 ; 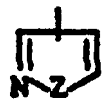 ; 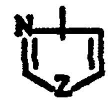 ;

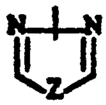 ; 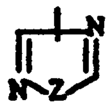 ; 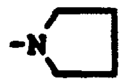 ; 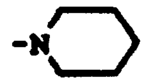 ;

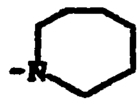 ; 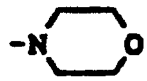 oder 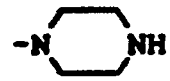

wobei Z jeweils für Sauerstoff oder Schwefel steht und wobei als Substituenten in Frage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl stehen, wobei als Phenylsubstituenten jeweils in Frage kommen:
Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl und Phenoxy oder

$R^3$ und $R^4$ gemeinsam mit dem Stickstoffatom, an welches sie gebunden sind, für einen gegebenenfalls ein- bis dreifach, gleich oder verschieden substituierten Heterocyclus der Formel

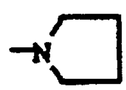 ; 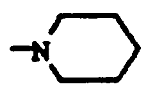 ; 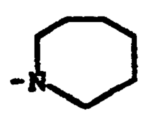 ; 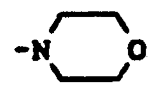 ;

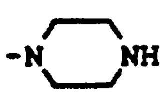 ; 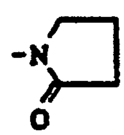 ; 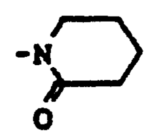 ; 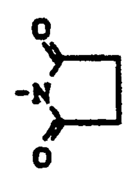 ;

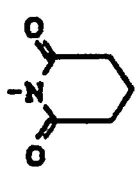 ; 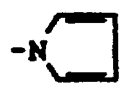 oder 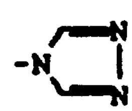 stehen,

wobei als Substituenten in Frage kommen: Methyl, Ethyl, n- oder i-Propyl, Chlor oder Trifluormethyl,

X für Sauerstoff oder Schwefel steht und

Y für Sauerstoff oder Schwefel steht, wobei

jedoch X nur dann für Schwefel steht, wenn mindestens einer der Reste $R^3$ oder $R^4$ nicht gleichzeitig für Wasserstoff, Methyl oder Ethyl steht.

**3.** Verfahren zur Herstellung von substituierten Triazolinonen der Formel (I)

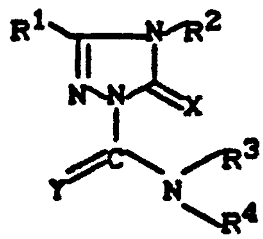

(I)

in welcher

$R^1$ für Wasserstoff, Alkyl, Alkenyl, Alkinyl, Halogenalkyl, Halogenalkenyl, Halogenalkinyl, Alkoxyalkyl, Alkoxy, für Cycloalkylalkyl, für Cycloalkyl oder für jeweils gegebenenfalls substituiertes Aralkyl oder Aryl steht,

$R^2$ für Alkyl, Alkenyl, Alkinyl, Halogenalkyl, Halogenalkenyl, Halogenalkinyl, Alkoxyalkyl, Alkoxy, für Cycloalkylalkyl, für Cycloalkyl oder für jeweils gegebenenfalls substituiertes Aralkyl oder Aryl steht,

$R^3$ und $R^4$ unabhängig voneinander jeweils für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkoximinoalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Aroyl oder Aryl, für Alkoxy, Alkenyloxy, Alkinyloxy, Aralkyloxy oder Aryloxy stehen oder gemeinsam mit dem Stickstoffatom, an welches sie gebunden sind, für einen gegebenenfalls substituierten Heterocyclus stehen,

X für Sauerstoff oder Schwefel steht und

Y für Sauerstoff oder Schwefel steht,

wobei jedoch X nur dann für Schwefel steht, wenn mindestens einer der Reste $R^3$ oder $R^4$ nicht gleichzeitig für Wasserstoff, Methyl oder Ethyl steht,

dadurch gekennzeichnet, daß man

a) 1-Chlor-(thio)carbonyltriazolinone der Formel (II),

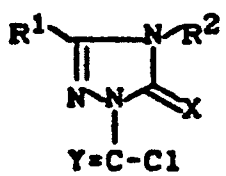

(II)

in welcher

$R^1$, $R^2$, X und Y die oben angegebene Bedeutung haben,

mit Aminen der Formel (III),

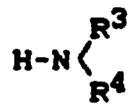

(III)

in welcher

$R^3$ und $R^4$ die oben angegebene Bedeutung haben,

gegebenenfalls in Gegenwart eines Verdünnungsmittels und gegebenenfalls in Gegenwart eines Säurebindemittels umsetzt, oder daß man

b) für den Fall, daß $R^3$ Wasserstoff bedeutet, wenn man in 1-Stellung unsubstituierte Triazolinone der Formel (IV),

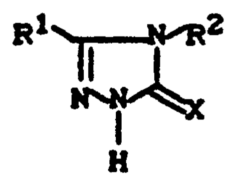

(IV)

in welcher

$R^1$, $R^2$ und X die oben angegebene Bedeutung haben,

mit Iso(thio)cyanaten der Formel (V),

$R^4$-N = C = T (V)

in welcher

$R^4$ und Y die oben angegebene Bedeutung haben,

gegebenenfalls in Gegenwart eines Verdünnungsmittels und gegebenenfalls in Gegenwart eines Reaktionshilfsmittels umsetzt.

**4.** Herbizide Mittel, gekennzeichnet durch einen Gehalt an mindestens einem substituierten Triazolinon der Formel (I) gemäß den Ansprüchen 1 und 3.

**5.** Verfahren zur Bekämpfung von Unkräutern, dadurch gekennzeichnet, daß man substituierte Triazolinone der Formel (I) gemäß den Ansprüchen 1 und 3 auf die Unkräuter und/oder ihren Lebensraum einwirken läßt.

**6.** Verwendung von substituierten Triazolinonen der Formel (I) gemäß den Ansprüchen 1 und 3 zur Bekämpfung von Unkräutern.

**7.** Verfahren zur Herstellung von herbiziden Mitteln, dadurch gekennzeichnet, daß man substituierte Triazolinone der Formel (I) gemäß Ansprüchen 1 und 3 mit Streckmitteln und/oder oberflächenaktiven Substanzen vermischt.

**8.** 1-Chlor-(thio)carbonyltriazolinone der Formel (II)

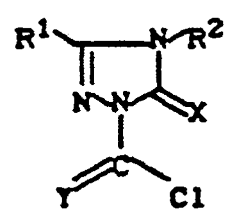

(II)

in welcher

$R^1$ für Wasserstoff, Alkyl, Alkenyl, Alkinyl, Halogenalkyl, Halogenalkenyl, Halogenalkinyl, Alkoxyalkyl, Alkoxy, für Cycloalkylalkyl, für Cycloalkyl oder für jeweils gegebenenfalls

substituiertes Aralkyl oder Aryl steht,

$R^2$ für Alkyl, Alkenyl, Alkinyl, Halogenalkyl, Halogenalkenyl, Halogenalkinyl, Alkoxyalkyl, Alkoxy, für Cycloalkylalkyl, für Cycloalkyl oder für jeweils gegebenenfalls substituiertes Aralkyl oder Aryl steht und

X und Y unabhängig voneinander für Sauerstoff oder Schwefel stehen.

**9.** Verfahren zur Herstellung von 1-Chlor-(thio)carbonyltriazolinonen der Formel (II),

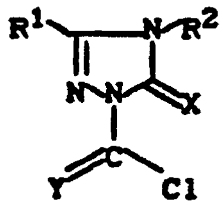

(II)

in welcher

$R^1$ für Wasserstoff, Alkyl, Alkenyl, Alkinyl, Halogenalkyl, Halogenalkenyl, Halogenalkinyl, Alkoxyalkyl, Alkoxy, für Cycloalkylalkyl, für Cycloalkyl oder für jeweils gegebenenfalls substituiertes Aralkyl oder Aryl steht,

$R^2$ für Alkyl, Alkenyl, Alkinyl, Halogenalkyl, Halogenalkyl, Halogenalkinyl, Alkoxyalkyl, Alkoxy, für Cycloalkylalkyl, für Cycloalkyl oder für jeweils gegebenenfalls substituiertes Aralkyl oder Aryl steht und

X und Y unabhängig voneinander für Sauerstoff oder Schwefel stehen,

dadurch gekennzeichnet, daß man in 1-Stellung unsubstituierte Triazolinone der Formel (IV),

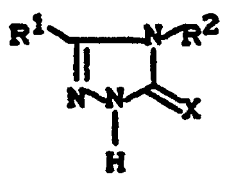

(IV)

in welcher

$R^1$, $R^2$ und X die oben angegebene Bedeutung haben,

mit (Thio)Phosgen der Formel (VI),

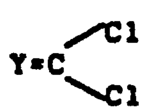

(VI)

in welcher

Y die oben angegebene Bedeutung hat,

gegebenenfalls in Gegenwart eines Verdünnungsmittel und gegebenenfalls in Gegenwart eines Säurebindemittels umsetzt.

**Claims**

1. Substituted triazolinones of the formula (I)

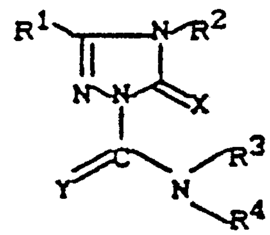

(I)

in which

R¹ represents hydrogen or represents straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, or alkoxyalkyl or alkoxy, each having 1 to 6 carbon atoms in the individual alkyl parts, or represents cycloalkylalkyl or cycloalkyl, each having 3 to 7 carbon atoms in the cycloalkyl part and optionally 1 to 6 carbon atoms in the straight-chain or branched alkyl part, or aralkyl or aryl, each having 6 to 10 carbon atoms in the aryl part and optionally 1 to 6 carbon atoms in the straight-chain or branched alkyl part, which are each optionally monosubstituted or polysubstituted by identical or different substituents, suitable substituents on aryl being in each case: halogen, cyano, nitro, and straight-chain or branched alkyl, alkoxy, alkylthio, halogenoalkyl, halogenoalkoxy or halogenoalkylthio, each having 1 to 4 carbon atoms and optionally 1 to 9 identical or different halogen atoms,

R² represents straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy, each having 1 to 6 carbon atoms in the individual alkyl parts, cycloalkylalkyl or cycloalkyl, each having 3 to 7 carbon atoms in the cycloalkyl part and optionally 1 to 6 carbon atoms in the straight-chain or branched alkyl part, or aralkyl or aryl, each having 6 to 10 carbon atoms in the aryl part and optionally 1 to 6 carbon atoms in the straight-chain or branched alkyl part, which are each optionally monosubstituted or polysubstituted by identical or different substituents, suitable substituents on aryl being in each case: halogen, cyano, nitro, and straight-chain or branched alkyl, alkoxy, alkylthio, halogenoalkyl, halogenoalkoxy or halogenoalkylthio, each having 1 to 4 carbon atoms and optionally 1 to 9 identical or different halogen atoms,

R³ and R⁴ independently of one another each represent hydrogen or represent straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, each having 2 to 8 carbon atoms and 1 to 15 or 13 identical or different halogen atoms respectively, cyanoalkyl having 1 to 8 carbon atoms, hydroxyalkyl having 1 to 8 carbon atoms and 1 to 6 hydroxyl groups, alkoxyalkyl, alkoximinoalkyl, alkoxycarbonylalkyl or alkoxycarbonylalkenyl, each having up to 6 carbon atoms in the individual alkyl or alkenyl parts, or alkylaminoalkyl or dialkylaminoalkyl, each having 1 to 6 carbon atoms in the individual alkyl parts, or represent cycloalkyl, cycloalkylalkyl, cycloalkenyl or cycloalkenylalkyl, each having 3 to 8 carbon atoms in the cycloalkyl or cycloalkenyl part and optionally 1 to 6 carbon atoms in the alkyl part, which are each optionally monosubstituted or polysubstituted by identical or different substituents, suitable substituents being in each case: halogen, cyano and straight-chain or branched alkyl or halogenoalkyl, each having 1 to 4 carbon atoms and optionally 1 to 9 identical or different halogen atoms or bivalent alkanediyl or alkenediyl, each having up to 4 carbon atoms; and in addition heterocyclylalkyl having 1 to 6 carbon atoms in the straightchain or branched alkyl part and 1 to 9 carbon atoms and also 1 to 3 heteroatoms - in particular nitrogen, oxygen and/or sulphur - in the heterocyclic part, which is optionally monosubstituted or polysubstituted in the heterocyclic part by identical or different substituents, suitable substituents being: halogen, cyano, nitro, and straight-chain or branched alkyl, alkoxy, alkylthio, halogenoalkyl, halogenoalkoxy, halogenoalkylthio or alkoxycarbonyl, each having 1 to 5 carbon atoms and optionally 1 to 9 identical or different halogen atoms; and in addition straight-chain or branched alkoxy having 1 to 8 carbon atoms, alkenyloxy having 2 to 8 carbon atoms or alkinyloxy having 2 to 8 carbon atoms, and finally aralkyl, aralkyloxy, aryloxy, aroyl or aryl, each having 6 to 10 carbon atoms in the aryl part and optionally 1 to 6 carbon atoms in the alkyl part, which are each optionally monosubstituted or polysubstituted by identical or different substituents, suitable substituents on aryl being in each case: halogen, cyano, nitro, hydroxyl, straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, each having 1 to 6 carbon atoms and optionally 1 to 9 identical or different halogen atoms, cycloalkyl having 3 to 6 carbon atoms and phenoxy and where, if appropriate, suitable substituents on alkyl are: halogen or cyano, or

$R^3$ and $R^4$, together with the nitrogen atom to which they are bonded, represent a five- to ten-membered heterocycle which can optionally contain 1 to 2 further heteroatoms, in particular nitrogen, oxygen and/or sulphur, and which is optionally monosubstituted or polysubstituted by identical or different substituents, suitable substituents being: halogen and straight-chain or branched alkyl or halogenoalkyl, each having 1 to 4 carbon atoms and optionally 1 to 9 identical or different halogen atoms and 1 to 2 oxo or thiono groups,

X represents oxygen or sulphur and

Y represents oxygen or sulphur,

where X only represents sulphur, however, when at least one of the radicals $R^3$ or $R^4$ does not simultaneously represent hydrogen, methyl or ethyl.

**2.** Substituted triazolinones of the formula (I) according to Claim 1 in which

$R^1$ represents hydrogen, methyl, ethyl, n- or i-propyl, n-, i-, s- or t-butyl, n- or i-pentyl, n- or i-hexyl, allyl, propargyl, methoxy, ethoxy, methoxymethyl, ethoxymethyl, methoxyethyl, ethoxyethyl, straight-chain or branched halogenoalkyl having 1 to 4 carbon atoms and 1 to 9 identical or different halogen atoms, cyclopentyl, cyclohexyl, cyclopropyl, cyclopropylmethyl, cyclohexylmethyl, cyclohexylethyl, or benzyl or phenyl, which are each optionally monosubstituted to trisubstituted by identical or different substituents, suitable substituents being in each case: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio,

$R^2$ represents methyl, ethyl, n- or i-propyl, n-, i-, s- or t-butyl, n- or i-pentyl, n- or i-hexyl, allyl, propargyl, methoxy, ethoxy, methoxymethyl, ethoxymethyl, methoxyethyl, ethoxyethyl, straight-chain or branched halogenoalkyl having 1 to 4 carbon atoms and 1 to 9 identical or different halogen atoms, cyclopentyl, cyclohexyl, cyclopropyl, cyclopropylmethyl, cyclohexylmethyl, cyclohexylethyl, or benzyl or phenyl, which are each optionally monosubstituted to trisubstituted by identical or different substituents, suitable substituents being in each case: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio,

$R^3$ and $R^4$ independently of one another each represent hydrogen, methyl, ethyl, n- or i-propyl, n-, i-, s- or t-butyl, n- or i-pentyl, n- or i-hexyl, n- or i-heptyl, n- or i-octyl, n- or i-nonyl, n- or i-decyl, n- or i-dodecyl, allyl, propenyl, n- or i-butenyl, n- or i-pentenyl, n- or i-hexenyl, propargyl, n- or i-butinyl, n- or i-pentinyl, n- or i-hexinyl, straight-chain or

branched halogenoalkyl having 1 to 4 carbon atoms and 1 to 9 identical or different halogen atoms, straight-chain or branched halogenoalkenyl or halogenoalkinyl, each having 3 to 5 carbon atoms and 1 to 3 halogen atoms, straight-chain or branched cyanoalkyl having 1 to 4 carbon atoms in the alkyl part, hydroxyalkyl having 1 to 6 carbon atoms and 1 to 3 hydroxyl groups, alkoxyalkyl, alkoximinoalkyl, alkoxycarbonylalkyl or alkoxycarbonylalkenyl, alkylaminoalkyl or dialkylaminoalkyl, each having up to 4 carbon atoms in the individual alkyl or alkenyl parts, or cyclopropyl, cyclopropylmethyl, cyclopropylethyl, cyclopentyl, cyclohexyl, cycloheptyl, cyclooctyl, cyclohexylmethyl, cyclohexylethyl, cyclohexenyl or cyclohexenylmethyl, which are each optionally monosubstituted to trisubstituted by identical or different substituents, suitable substituents in the cyclic and where appropriate in the aliphatic part being in each case: fluorine, chlorine, bromine, methyl, ethyl, n-or i-propyl, n-, L-, s- or t-butyl, cyano, methanediyl, ethanediyl, butanediyl or butadienediyl; and additionally heterocyclylmethyl, heterocyclylethyl or heterocyclylpropyl, which are optionally monosubstituted to trisubstituted in the heterocyclic part by identical or different substituents, suitable heterocycles being in each case:

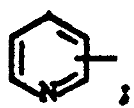 ; 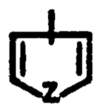 ; 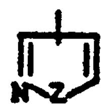 ; 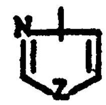 ;

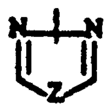 ; 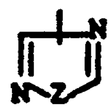 ; 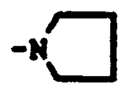 ; 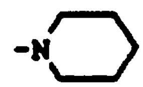 ;

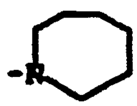 ; 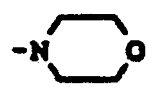 or 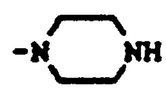

where Z in each case represents oxygen or sulphur and where suitable substituents are: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio; and in addition straight-chain or branched alkoxy having 1 to 6 carbon atoms, alkenyloxy having 3 to 6 carbon atoms or alkinyloxy having 3 to 6 carbon atoms, or optionally straight-chain or branched benzyl, phenylethyl, phenylpropyl, phenylbutyl, phenylpentyl, phenylhexyl, phenylheptyl, phenylcyanomethyl, phenylcyanoethyl, phenylcyanopropyl, benzyloxy, phenylethoxy, phenoxy, benzoyl, phenyl or naphthyl, which are each optionally monosubstituted to trisubstituted by identical or different substituents, suitable substituents on phenyl being in each case: fluorine, chlorine, bromine, hydroxyl, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl,methylsulphinyl, methylsulphonyl, acetyl, propionyl, methoxycarbonyl, ethoxycarbonyl, cyclohexyl and phenoxy or

$R^3$ and $R^4$, together with the nitrogen atom to which they are bonded, represent a heterocycle of the formula

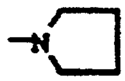 ; 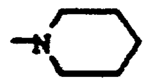 ; 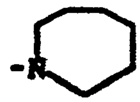 ; 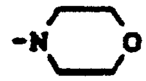 ;

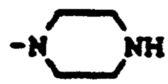 , 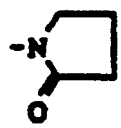 ; 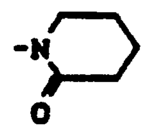 ; 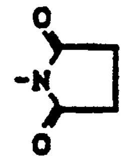 ;

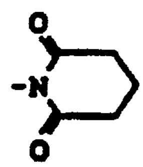 ; 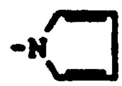 or 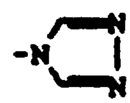

which is optionally monosubstituted to trisubstituted by identical or different substituents, suitable substituents being: methyl, ethyl, n-or i-propyl, chlorine or trifluoromethyl,

X represents oxygen or sulphur and

Y represents oxygen or sulphur,

where X only represents sulphur, however, when at least one of the radicals $R^3$ or $R^4$ does not simultaneously represent hydrogen, methyl or ethyl.

**3.** Process for the preparation of substituted triazolinones of the formula (I)

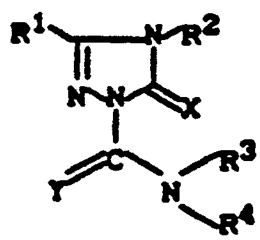

(I)

in which

$R^1$ represents hydrogen, alkyl, alkenyl, alkinyl, halogenoalkyl, halogenoalkenyl, halogenoalkinyl, alkoxyalkyl, alkoxy, cycloalkylalkyl, cycloalkyl or optionally substituted aralkyl or aryl,

$R^2$ represents alkyl, alkenyl, alkinyl, halogenoalkyl, halogenoalkenyl, halogenoalkinyl, alkoxyalkyl, alkoxy, cycloalkylalkyl, cycloalkyl or optionally substituted aralkyl or aryl,

$R^3$ and $R^4$ independently of one another each represent hydrogen, alkyl, alkenyl, alkinyl, halogenoalkyl, halogenoalkenyl, halogenoalkinyl, cyanoalkyl, hydroxyalkyl, alkoxyalkyl, alkoximinoalkyl, alkoxycarbonylalkyl, alkoxycarbonylalkenyl, alkylaminoalkyl, dialkylaminoalkyl, optionally substituted cycloalkyl, cycloalkylalkyl, cycloalkenyl or cycloalkenylalkyl, optionally substituted heterocyclylalkyl, optionally substituted aralkyl, aroyl or aryl, alkoxy, alkenyloxy, alkinyloxy, aralkyloxy or aryloxy or, together with the nitrogen atom to which they are bonded, represent an optionally substituted heterocycle,

X represents oxygen or sulphur and

Y represents oxygen or sulphur,

where X only represents sulphur, however, when at least one of the radicals $R^3$ or $R^4$ does not simultaneously represent hydrogen, methyl or ethyl,

characterised in that

a) 1-chloro-(thio)carbonyltriazolinones of the formula (II)

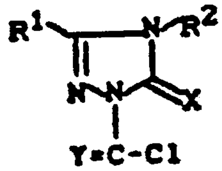

(II)

in which

$R^1$, $R^2$, X and Y have the abovementioned meaning, are reacted with amines of the formula (III)

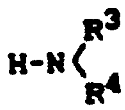

(III)

in which

$R^3$ and $R^4$ have the abovementioned meaning, if desired in the presence of a diluent and if desired in the presence of an acid-binding agent, or in that

b) in the case where $R^3$ denotes hydrogen, 1-unsubstituted triazolinones of the formula (IV)

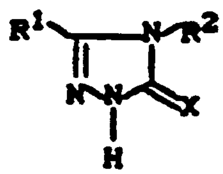

(IV)

in which

$R^1$, $R^2$ and X have the abovementioned meaning, are reacted with iso(thio)cyanates of the formula (V)

$R^4$-N=C=Y (V)

in which

$R^4$ and Y have the abovementioned meaning, if desired in the presence of a diluent and if desired in the presence of a reaction auxiliary.

**4.** Herbicidal agents, characterised in that they contain at least one substituted triazolinone of the formula (I) according to Claims 1 and 3.

**5.** Process for combating weeds, characterised in that substituted triazolinones of the formula (I) according to Claims 1 and 3 are allowed to act on the weeds and/or their environment.

**6.** Use of substituted triazolinones of the formula (I) according to Claims 1 and 3 for combating weeds.

**7.** Process for the preparation of herbicidal agents, characterised in that substituted triazolinones of the formula (I) according to Claims 1 and 3 are mixed with extenders and/or surface-active substances.

**8.** 1-Chloro-(thio)carbonyltriazolinones of the formula (II)

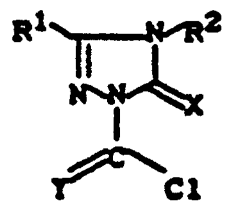

(II)

in which

R¹ represents hydrogen, alkyl, alkenyl, alkinyl, halogenoalkyl, halogenoalkenyl, halogenoalkinyl, alkoxyalkyl, alkoxy, cycloalkylalkyl, cycloalkyl or optionally substituted aralkyl or aryl,

R² represents alkyl, alkenyl, alkinyl, halogenoalkyl, halogenoalkenyl, halogenoalkinyl, alkoxyalkyl, alkoxy, cycloalkylalkyl, cycloalkyl or optionally substituted aralkyl or aryl, and

X and Y independently of one another represent oxygen or sulphur.

**9.** Process for the preparation of 1-chloro(thio)-carbonyltriazolinones of the formula (II)

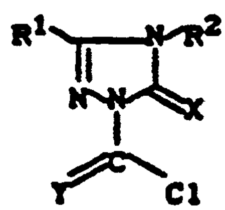

(II)

in which

$R^1$ represents hydrogen, alkyl, alkenyl, alkinyl, halogenoalkyl, halogenoalkenyl, halogenoalkinyl, alkoxyalkyl, alkoxy, cycloalkylalkyl, cycloalkyl or optionally substituted aralkyl or aryl,

$R^2$ represents alkyl, alkenyl, alkinyl, halogenoalkyl, halogenoalkenyl, halogenoalkinyl, alkoxyalkyl, alkoxy, cycloalkylalkyl, cycloalkyl or optionally substituted aralkyl or aryl, and

X and Y independently of one another represent oxygen or sulphur,

characterised in that 1-unsubstituted triazolinones of the formula (IV)

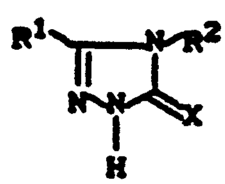

(IV)

in which

$R^1$, $R^2$ and X have the abovementioned meaning, are reacted with (thio)phosgene of the formula (VI)

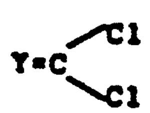

(VI)

in which

Y has the abovementioned meaning, if desired in the presence of a diluent and if desired in the presence of an acid-binding agent.

**Revendications**

1. Triazolinones substituées de formule (I) :

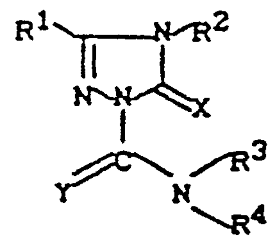

(I)

dans laquelle

$R^1$ représente un atome d'hydrogène, un groupe chaque fois linéaire ou ramifié, alkyle ayant 1 à 8 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène(s), identiques ou différents, halogéno-alcényle ayant 2 à 8 atomes de carbone et 1 à 15 atomes d'halogène(s) identiques ou différents, halogéno-alcynyle comportant 2 à 8 atomes de carbone et 1 à 13 atomes d'halogène(s) identiques ou différents, alcoxyalkyle ou alcoxy ayant chacun 1 à 6 atomes de carbone dans les parties alkyle, cycloalkylalkyle ou cycloalkyle ayant chacun 3 à 7 atomes de carbone dans la partie cycloalkyle et éventuellement 1 à 6 atomes de carbone dans la partie alkyle linéaire ou ramifiée, ou un groupe à chaque fois éventuellement substitué une ou plusieurs fois, de manière identique ou différente, aralkyle ou aryle ayant chacun 6 à 10 atomes de carbone dans la partie aryle et éventuellement 1 à 6 atomes de carbone dans la partie alkyle linéaire ou ramifiée, et l'on peut citer comme substituant fixé sur la partie aryle : un atome d'halogène, un reste cyano, nitro, ainsi que éventuellement un reste à chaque fois linéaire ou ramifié, alkyle, alcoxy, alkylthio, halogéno-alkyle, halogéno-alcoxy ou halogéno-alkylthio ayant chacun 1 à 4 atomes de carbone et éventuellement 1 à 9 atomes d'halogène(s), identiques ou différents,

$R^2$ représente un groupe, à chaque fois linéaire ou ramifié, alkyle ayant 1 à 8 atomes de carbone, alcényle ayant 2 à 8 atomes de carbone, alcynyle comportant 2 à 8 atomes de carbone, halogéno-alkyle ayant 1 à 8 atomes de carbone et 1 à 17 atomes d'halogène(s) identiques ou différents, halogéno-alcényle ayant 2 à 8 atomes de carbone et 1 à 15 atomes d'halogène(s) identiques ou différents, halogéno-alcynyle ayant 2 à 8 atomes de carbone et 1 à 13 atomes d'halogène(s) identiques ou différents, alcoxyalkyle ou alcoxy comportant à chaque fois 1 à 6 atomes de carbone dans les parties alkyles, cycloakylalkyle ou cycloalkyle ayant à chaque fois 3 à 7 atomes de carbone dans la partie cycloalkyle et éventuellement 1 à 6 atomes de carbone dans la partie alkyle linéaire ou ramifiée, ou bien un groupe, à chaque fois éventuellement substitué une ou plusieurs fois, de manière identique ou différente, aralkyle ou aryle ayant chacun 6 à 10 atomes de carbone dans la partie aryle et éventuellement 1 à 6 atomes de carbone dans la partie alkyle linéaire ou ramifiée, et l'on peut citer à chaque fois comme substituant fixé sur la partie aryle : un atome d'halogène, un reste cyano, nitro, ainsi qu'un reste à chaque fois linéaire ou ramifié, alkyle, alcoxy, alkylthio, halogéno-alkyle, halogéno-alcoxy ou halogéno-alkylthio ayant chacun 1 à 4 atomes de carbone et éventuellement 1 à 9 atomes d'halogène(s) identiques ou différents,

$R^3$ et $R^4$ représentent chacun, indépendamment l'un de l'autre un atome d'hydrogène, un groupe, à chaque fois linéaire ou ramifié, alkyle ayant 1 à 18 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène(s) identiques ou différents, halogéno-alcényle ou halogéno-alcynyle ayant à chaque fois 2 à 8 atomes de carbone et 1 à 15 ou 13 atomes d'halogène(s) identiques ou différents, cyano-alkyle ayant 1 à 8 atomes de carbone, hydroxyalkyle ayant 1 à 8 atomes de carbone et 1 à 6 groupes hydroxy, alcoxyalkyle, alcoxyiminoalkyle, alcoxycarbonylalkyle ou alcoxycarbonylalcényle ayant chacun jusqu'à 6 atomes de carbone dans la

partie alkyle ou alcényle, alkylaminoalkyle ou dialkylaminoalkyle ayant chacun l à 6 atomes de carbone dans la partie alkyle ou un groupe, à chaque fois éventuellement substitué une ou plusieurs fois de manière identique ou différente, cycloalkyle, cycloalkylalkyle, cycloalcényle ou cycloalcénylalkyle ayant chacun 3 à 8 atomes de carbone dans la partie cycloakyle ou cycloalcényle et éventuellement 1 à 6 atomes de carbone dans la partie alkyle et, l'on peut citer à chaque fois comme substituant : un atome d'halogène, un reste cyano ainsi qu'un reste à chaque fois linéaire ou ramifié, alkyle ou halogéno-alkyle ayant chacun 1 à 4 atomes de carbone et éventuellement 1 à 9 atomes d'halogène(s) identiques ou différents ou un groupe alcanediyle ou alcènediyle chacun présentant deux liaisons et comportant chacun jusqu'à 4 atomes de carbone ; en outre $R^3$ et $R^4$ peuvent représenter un groupe hétérocyclylalkyle (éventuellement substitué une ou plusieurs fois, de façon identique ou différente dans la partie hétérocyclyle) et comportant 1 à 4 atomes de carbone dans la partie alkyle linéaire ou ramifiée et 1 à 9 atomes de carbone ainsi que 1 à 3 hétéroatomes - en particulier l'azote, l'oxygène et/ou le soufre - dans la partie hétérocyclyle, et l'on peut citer comme substituant : un reste halogéno, cyano, nitro, ainsi qu'un reste à chaque fois linéaire ou ramifié, alkyle, alcoxy, alkylthio, halogéno-alkyle, halogéno-alcoxy, halogéno-alkylthio ou alcoxycarbonyle ayant chacun l à 5 atomes de carbone et éventuellement 1 à 9 atomes d'halogène(s) identiques ou différents ; en outre $R^3$ et $R^4$ peuvent représenter chacun un groupe, à chaque fois linéaire ou ramifié, alcoxy ayant 1 à 8 atomes de carbone, alcényloxy ayant 2 à 8 atomes de carbone ou alcynyloxy ayant 2 à 8 atomes de carbone et enfin un groupe, à chaque fois éventuellement substitué une ou plusieurs fois de manière identique ou différente, aralkyle, aralkyloxy, aryloxy, aroyle ou aryle ayant à chaque fois 6 à 10 atomes de carbone dans la partie aryle et éventuellement 1 à 6 atomes de carbone dans la partie alkyle, et l'on peut citer à chaque fois comme substituant fixé sur la partie aryle : un atome d'halogène, un reste cyano, nitro, hydroxy, un reste à chaque fois linéaire ou ramifié, alkyle, alcoxy, alkylthio, halogéno-alkyle, halogéno-alcoxy, halogéno-alkylthio, alkylsulfynyle, alkylsulfonyle, halogéno-alkylsulfynyle, halogéno-alkylsulfonyle, alcanoyle ou alcoxycarbonyle ayant chacun 1 à 6 atomes de carbone et éventuellement 1 à 9 atomes d'halogène(s) identiques ou différents, cycloalkyle comportant 2 à 6 atomes de carbone et phénoxy et, comme substituant fixé sur la partie alkyle, éventuellement un reste halogéno ou cyano, ou bien

$R^3$ et $R^4$, pris avec l'atome d'azote auquel ils sont liés, représentent un hétérocycle comportant 5 à 10 chaînons, éventuellement substitué une ou plusieurs fois de manière identique ou différente, qui peut contenir éventuellement 1 à 2 autres hétéroatomes, en particulier l'azote, l'oxygène et/ou le soufre, et l'on peut citer comme substituants : un atome d'halogène ainsi qu'un reste chaque fois linéaire ou ramifié, alkyle ou halogéno-alkyle ayant chacun 1 à 4 atomes de carbone et comportant éventuellement 1 à 9 atomes d'halogène(s), identiques ou différents, ainsi que 1 à 2 groupes oxo ou thiono,

X représente un atome d'oxygène ou de soufre, et

Y représente un atome d'oxygène ou de soufre,

mais X ne représente un atome de soufre que lorsqu'au moins l'un des restes $R^3$ ou $R^4$ ne représente pas simultanément un atome d'hydrogène ou un groupe méthyle ou éthyle.

**2.** Triazolinones substituées de formule (I) selon la revendication 1, dans lesquelles :

$R^1$ représente un atome d'hydrogène, un groupe méthyle, éthyle, n- ou i-propyle, n-, i-, s- ou t-butyle, n- ou i-pentyle, n- ou i-hexyle, un groupe allyle, propargyle, méthoxy, éthyoxy, méthoxyméthyle, éthoxyméthyle, méthoxyéthyle, éthoxyéthyle, un groupe halogéno-alkyle linéaire ou ramifié et comportant 1 à 4 atomes de carbone et 1 à 9 atomes d'halogène(s) identiques ou différents, un groupe cyclopentyle, cyclohexyle, cyclopropyle, cyclopropylméthyle, cyclohexylméthyle, cyclohexyléthyle ou un groupe éventuellement substitué 1 à 3 fois de manière identique ou différente, benzyle ou phényle, et l'on peut alors citer à chaque fois comme substituants : un atome de fluor, de chlore ou de brome, un reste cyano, nitro, méthyle, éthyle, n- ou i-propyle, n-, i-, s- ou t-butyle, méthoxy, éthoxy, méthylthio, trifluorométhyle, trifluorométhoyx ou trifluorométhylthio,

$R^2$ représente un groupe méthyle, éthyle, n- ou i-propyle, n-, i-, s- ou t-butyle, n- ou i-pentyle, n- ou i-hexyle, un groupe allyle, propargyle, méthoxy, éthoxy, méthoxyméthyle, éthoxyméthyle, méthoxyéthyle, éthoxyéthyle, un groupe halogéno-alkyle linéaire ou ramifié comportant 1 à 4 atomes de carbone et 1 à 9 atomes d'halogène(s), identiques ou différents, un groupe cyclopentyle, cyclohexyle, cyclopropyle, cyclopropylméthyle, cyclohexylméthyle, cyclohexyléthyle ou un groupe à chaque fois éventuellement substitué 1 à 3 fois, de manière identique ou différente, benzyle ou phényle et l'on peut alors citer comme substituant à chaque fois : un atome de fluor, de chlore ou de brome, un reste cyano, nitro, méthyle, éthyle, n- ou i-propyle, n-, i-, s- ou t-butyle, méthoxy, éthoxy, méthylthio, trifluorométhyle, trifluorométhoxy ou trifluorométhylthio,

$R^3$ et $R^4$ représentent chaoun, indépendamment l'un de l'autre, un atome d'hydrogène, un groupe méthyle, éthyle, n- ou i-propyle, n-, i-, s- ou t-butyle, n- ou i-pentyle, n- ou i-hexyle, n- ou i-heptyle, n- ou i-octyle, n- ou i-nonyle, n- ou i-décyle, n- ou i-dodécyle, un groupe allyle, propényle, n- ou i-butényle, n- ou i-pentényle, n- ou i-hexényle, propargyle, n- ou i-butynyle, n- ou i-pentynyle, n- ou i-hexynyle, un groupe halogéno-alkyle linéaire ou ramifié comportant 1 à 4 atomes de carbone et 1 à 9 atomes d'halogène(s) identiques ou différents, un groupe à chaque fois linéaire ou ramifié, halogéno-alcényle ou halogéno-alcynyle ayant chacun 3 à 5 atomes de carbone et 1 à 3 atomes d'halogène, un groupe, chaque fois linéaire ou ramifié, cyano-alkyle ayant 1 à 4 atomes de carbone dans la partie alkyle, hydroxyalkyle ayant 1 à 6 atomes de carbone et 1 à 3 groupes hydroxyles, alcoxyalkyle, alcoxyiminoalkyle, alcoxycarbonylalkyle ou alcoxycarbonylalcényle, alkylaminoalkyle ou dialkylaminoalkyle ayant chacun jusqu'à 4 atomes de carbone dans les parties alkyles ou alcényles, ou un groupe, à chaque fois éventuellement substitué 1 à 3 fois de manière identique ou différente, cyclopropyle, cyclopropylméthyle, cyclopropyléthyle, cyclopentyle, cyclohexyle, cycloheptyle, cyclooctyle, cyclohexylméthyle, cyclohexyléthyle, cyclohexényle ou cyclohexénylméthyle, et l'on peut citer comme substituant fixé dans la partie cyclique et éventuellement dans la partie aliphatique, à chaque fois : un atome de fluor, de chlore ou de brome, un groupe méthyle, éthyle, n- ou i-propyle, n-, i-, s- ou t-butyle, cyano, méthanediyle, éthanediyle, butanediyle ou butadiènediyle ;

en outre un groupe, éventuellement substitué 1 à 3 fois, de manière identique ou différente dans la partie hétérocyclyle, hétérocyclylméthyle, hétérocyclyléthyle ou hétérocyclylpropyle, et l'on peut citer à chaque fois comme hétérocycles :

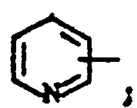 ; 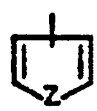 ; 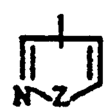 ; 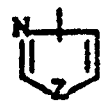 ;

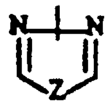 ; 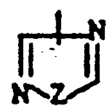 ; 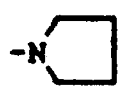 ; 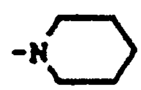 ;

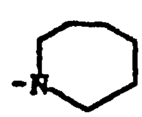 ; 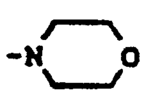 ou 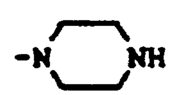

où Z représente à chaque fois un atome d'oxygène ou de soufre et l'on peut citer comme substituants :

un atome de fluor, de chlore, de brome, un reste cyano, nitro, méthyle, éthyle, n- ou i-propyle, n-, i-, s- ou t-butyle, méthoxy, éthoxy, méthylthio, trifluorométhyle, trifluorométhoxy ou trifluorométhylthio ;

en outre, $R^3$ et $R^4$ peuvent représenter un groupe, à chaque fois linéaire ou ramifié, alcoxy ayant 1 à 6 atomes de carbone, alcényloxy ayant 3 à 6 atomes de carbone ou alcynyloxy ayant 3 à 6 atomes de carbone ou un groupe, à chaque fois éventuellement substitué 1 à 3 fois, de manière identique ou différente, comportant éventuellement une partie alkyle linéaire ou ramifiée, benzyle, phényléthyle, phénylpropyle, phénylbutyle, phénylpentyle, phénylhexyle, phénylheptyle, phénylcyanométhyle, phénylcyanéthyle, phénylcyanopropyle, benzyloxy, phényléthyloxy, phénoxy, benzoyle, phényle ou naphtyle et l'on peut citer à chaque fois comme substituant fixé sur le noyau phényle :

un atome de fluor, de chlore ou de brome, un reste hydroxy, cyano, nitro, méthyle, éthyle, n- ou i-propyle, n-, i-, s- ou t-butyle, méthoxy, éthoxy, méthylthio, trifluorométhyle, trifluorométhoxy, trifluorométhylthio, trifluorométhylsulfinyle, trifluorométhylsulfonyle, méthylsulfinyle, méthylsulfonyle, acétyle, propionyle, méthoxycarbonyle, éthoxycarbonyle, cyclohexyle ou phénoxy ou bien

$R^3$ et $R^4$ pris avec l'atome d'azote auquel ils sont liés, représentent un hétérocycle, éventuellement substitué une à plusieurs fois, de manière identique ou différente, et répondant à la formule :

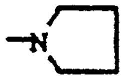 ; 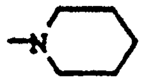 ; 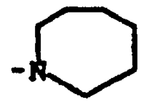 ; 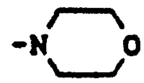 ;

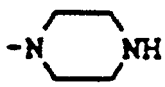 ; 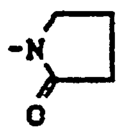 ; 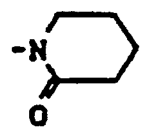 ; 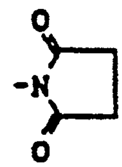 ;

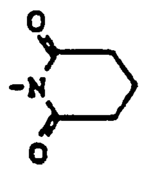 ; 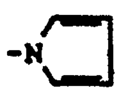 ou 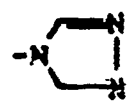

et l'on peut citer comme subsituants : un reste méthyle, éthyle, n- ou i-propyle, chloro ou trifluorométhyle,

X représente un atome d'oxygène ou de soufre, et

Y représente un atome d'oxygène ou de soufre,

mais X ne peut représenter un atome de soufre que lorsqu'au moins l'un des restes $R^3$ ou $R^4$ ne représente pas simultanément un atome d'hydrogène ou un groupe méthyle ou éthyle.

3. Procédé pour préparer des triazolinones substituées, de formule (I) :

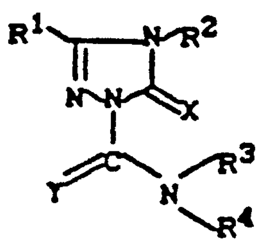

(I)

dans laquelle

$R^1$ représente un atome d'hydrogène, un groupe alkyle, alcényle, alcynyle, halogéno-alkyle, halogéno-alcényle, halogéno-alcynyle, alcoxyalkyle, alcoxy, cycloalkylalkyle, cycloalkyle ou un groupe (à chaque fois éventuellement substitué) aralkyle ou aryle,

$R^2$ représente un groupe alkyle, alcényle, alcynyle, halogéno-alkyle, halogéno-alcényle, halogéno-alcynyle, alcoxyalkyle, alcoxy, un groupe cycloalkylalkyle, cycloalkyle ou un groupe (éventuellement substitué) aralkyle ou aryle,

$R^3$ et $R^4$ représentent chacun indépendamment l'un de l'autre un atome d'hydrogène, un groupe alkyle, alcényle, alcynyle, halogéno-alkyle, halogéno-alcényle, halogéno-alcynyle, cyanalkyle, hydroxyalkyle, alcoxyalkyle, alcoxyiminoalkyle, alcoxycarbonylalkyle, alcoxycarbonylalcényle, alkylaminoalkyle, dialkylaminoalkyle, un groupe (éventuellement substitué) cycloalkyle, cycloaklylalkyle, cycloalcényle ou cycloalcénylalkyle, un groupe hétérocyclylalkyle éventuellement substitué, un groupe (chacun éventuellement substitué) aralkyle, aroyle ou aryle, un groupe alcoxy, alcényloxy, alcynyloxy, aralkyloxy ou aryloxy, ou ils forment, avec l'atome d'azote auquel ils sont reliés, un hétérocycle éventuellement substitué,

X représente un atome d'oxygène ou de soufre, et

Y représente un atome d'oxygène ou de soufre,

X ne représentant cependant un atome de soufre que lorsqu'au moins l'un des restes $R^3$, $R^4$ ne représente pas simultanément un atome d'hydrogène ou un groupe méthyle ou éthyle,

procédé caractérisé en ce que :

a) on fait réagir des 1-chloro-(thio)carbonyltriazolinones de formule (II) :

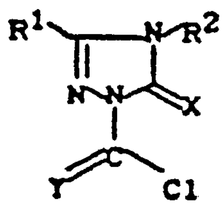

(II)

dans laquelle

$R^1$ $R^2$ X et Y ont le sens précité,

avec des amines de formule (III) :

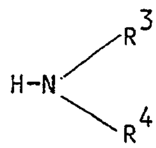

(III)

dans laquelle

$R^3$ et $R^4$ ont le sens indiqué ci-dessus,

en opérant éventuellement en présence d'un diluant et éventuellement d'un agent de fixation des acides, ou bien en ce que :

b) au cas où $R^3$ représente un atome d'hydrogène, on fait réagir des triazolinones non substituées en position 1 et répondant à la formule (IV) :

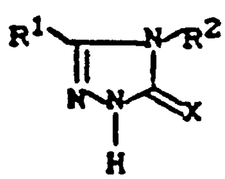

(IV)

dans laquelle

$R^1$ , $R^2$ et X ont le sens indiqué ci-dessus,

avec des iso(thio)cyanates de formule (V) :

$R^4$-N=C=Y (V)

dans laquelle

$R^4$ et Y ont le sens indiqué ci-dessus,

en opérant éventuellement en présence d'un diluant et éventuellement en présence d'un adjuvant de la réaction.

4. Produit herbicide, caractérisé en ce qu'il contient au moins une triazolinone substituée, répondant à la formule (I) selon les revendications 1 et 3.

5. Procédé pour lutter contre les mauvaises herbes, caractérisé en ce qu'on fait agir des triazolinones substituées, de formule (I) selon les revendications 1 et 3, sur les mauvaises herbes et/ou sur leur biotope ou espace vital.

6. Utilisation de triazolinones substituées de formule (I) selon les revendications 1 et 3, pour combattre des mauvaises herbes.

7. Procédé pour préparer des produits herbicides, caractérisé en ce qu'on mélange des triazolinones substituées, de formule (I) selon les revendications 1 et 3, avec des agents d'allongement et/ou des substances tensio-actives.

8. 1-chloro-(thio)carbonyltriazolinones de formule (II) :

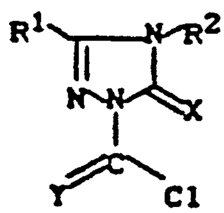

(II)

dans laquelle

$R^1$ représente un atome d'hydrogène, un groupe alkyle, alcényle, alcynyle, halogéno-alkyle, halogéno-alcényle, halogéno-alcynyle, alcoxyalkyle, alcoxy, cycloalkylalkyle, cycloalkyle ou un groupe à chaque fois éventuellement substitué, aralkyle ou aryle,

$R^2$ représente un groupe alkyle, alcényle, alcynyle, halogéno-alkyle, halogéno-alcényle, halogéno-alcynyle, alcoxyalkyle, alcoxy, cycloalkylalkyle, cycloalkyle ou un groupe, éventuellement chacun substitué, aralkyle ou aryle et

X et Y représentent chacun indépendamment l'un de l'autre, un atome d'oxygène ou de soufre.

9. Procédé pour préparer des 1-chloro-(thio)carbonyltriazolinones de formule (II) :

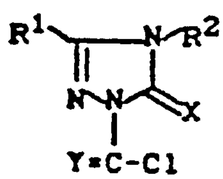

(II)

dans laquelle

$R^1$ représente un atome d'hydrogène, un groupe alkyle, alcényle, alcynyle, halogéno-alkyle, halogéno-alcényle, halogéno-alcynyle, alcoxyalkyle, alcoxy, cycloalkylalkyle, cycloalkyle ou un groupe, à chaque fois éventuellement substitué, aralkyle ou aryle,

$R^2$ représente un groupe alkyle, alcényle, alcynyle, halogéno-alkyle, halogéno-alcényle, halogéno-alcynyle, alcoxyalkyle, alcoxy, cycloalkylalkyle, cycloalkyle ou un groupe éventuellement chacun substitué, aralkyle ou aryle et

X et Y représentent chacun, indépendamment l'un de l'autre, un atome d'oxygène ou de soufre,

procédé caractérisé en ce qu'on fait réagir des triazolinones, non substituées en position 1 et répondant à la formule (IV) :

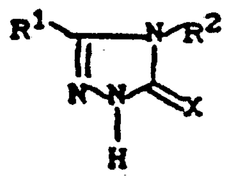

(IV)

dans laquelle

$R^1$ $R^2$ et X ont te sens précité,

avec du (thio)phosgène de formule (VI) :

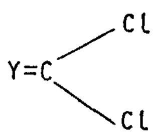

(VI)

dans laquelle

Y a le sens précité,

en opérant éventuellement en présence d'un diluant et éventuellement en présence d'un agent de fixation des acides.